(19) Europäisches Patentamt
European Patent Office
Office européen des brevets

(11) Veröffentlichungsnummer: **0 446 743 B1**

(12) # EUROPÄISCHE PATENTSCHRIFT

(45) Veröffentlichungstag der Patentschrift: **02.08.95**

(51) Int. Cl.$^6$: **C07D 239/47**, A01N 47/36, C07D 239/52, C07D 239/48

(21) Anmeldenummer: **91103181.3**

(22) Anmeldetag: **02.03.91**

Die Akte enthält technische Angaben, die nach dem Eingang der Anmeldung eingereicht wurden und die nicht in dieser Patentschrift enthalten sind.

(54) **Herbizide Sulfonylharnstoffe, Verfahren und Zwischenprodukte zu ihrer Herstellung.**

(30) Priorität: **10.03.90 DE 4007683**

(43) Veröffentlichungstag der Anmeldung:
**18.09.91 Patentblatt 91/38**

(45) Bekanntmachung des Hinweises auf die Patenterteilung:
**02.08.95 Patentblatt 95/31**

(84) Benannte Vertragsstaaten:
**AT BE CH DE ES FR GB IT LI NL**

(56) Entgegenhaltungen:
**EP-A- 0 084 020**
**EP-A- 0 169 815**

(73) Patentinhaber: **BASF Aktiengesellschaft**
**Carl-Bosch-Strasse 38**
**D-67063 Ludwigshafen (DE)**

(72) Erfinder: **Hamprecht, Gerhard, Dr.**
**Rote-Turm-Strasse 28**
**W-6940 Weinhein (DE)**
Erfinder: **Mayer, Horst, Dr.**
**Faselwiese 19**
**W-6700 Ludwigshafen (DE)**
Erfinder: **Westphalen, Karl-Otto, Dr.**
**Mausbergweg 58**
**W-6720 Speyer (DE)**
Erfinder: **Wuerzer, Bruno, Dr.**
**Ruedigerstrasse 13**
**W-6701 Otterstadt (DE)**
Erfinder: **Gerber, Matthias, Dr.**
**Ritterstrasse 3**
**W-6704 Mutterstadt (DE)**
Erfinder: **Grossmann, Klaus, Dr.**
**Wilhelm-Busch-Strasse 5**
**W-6703 Limburgerhof (DE)**
Erfinder: **Rademacher, Wilhelm, Dr.**
**Austrasse 1**
**W-6703 Limburgerhof (DE)**

EP 0 446 743 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte Sulfonylharnstoffe der allgemeinen Formel I,

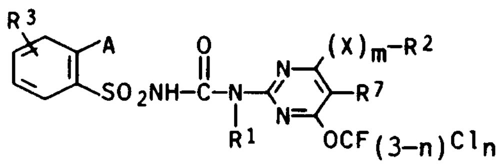

I

in der n und m für 0 oder 1 stehen und die Substituenten folgende Bedeutung haben:

- $R^1$ Wasserstoff, $C_1$-$C_4$-Alkyl, $C_3$-$C_6$-Alkenyl oder $C_3$-$C_6$-Alkinyl;
- $R^2$ Halogen oder Trifluormethyl, wenn m für 0 steht oder $C_1$-$C_4$-Alkyl, $C_3$-$C_6$-Alkenyl oder $C_3$-$C_6$-Alkinyl, wenn m 1 bedeutet oder Trifluor-oder Chlordifluormethyl wenn X für 0 oder S und m für 1 steht;
- X 0, S oder N-$R^4$, wobei $R^4$ für Wasserstoff oder $C_1$-$C_4$-Alkyl steht;
- $R^3$ Wasserstoff, Halogen, $C_1$-$C_4$-Alkyl, $C_1$-$C_4$-Halogenalkyl oder $C_1$-$C_4$-Alkoxy;
- A $C_1$-$C_4$-Halogenalkyl, ein Halogenatom oder ein Rest,

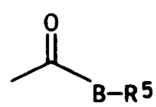

wobei

- B ein Sauerstoffatom oder eine Alkyliminogruppe $NR^6$;
- $R^5$ Wasserstoff, eine $C_1$-$C_6$-Alkylgruppe, welche bis zu drei der folgenden Reste tragen kann: Halogen, $C_1$-$C_4$-Alkoxy, $C_1$-$C_4$-Alkylthio, $C_1$-$C_4$-Halogenalkoxy, $C_1$-$C_4$-Alkoxy-$C_1$-$C_2$-alkoxy, $C_3$-$C_7$-Cycloalkyl und/oder Phenyl; eine $C_5$-$C_7$-Cycloalkylgruppe, welche bis zu drei $C_1$-$C_4$-Alkylgruppen tragen kann; eine $C_3$-$C_6$-Alkenylgruppe oder eine $C_3$-$C_6$-Alkinylgruppe;
- $R^6$ Wasserstoff, eine $C_1$-$C_6$-Alkylgruppe, oder gemeinsam mit $R^5$ eine $C_4$-$C_6$-Alkylenkette, worin eine Methylengruppe durch ein Sauerstoffatom oder eine $C_1$-$C_4$-Alkyliminogruppe ersetzt sein kann, bedeutet;
- $R^7$ Wasserstoff oder Halogen.

Des weiteren betrifft die Erfindung ein Verfahren zur Herstellung der Verbindungen I sowie ihre Verwendung als herbizide Mittel und Zwischenprodukte zur Herstellung der Sulfonylharnstoffe I.

Aus der US-A-4547215 sind verschiedene Sulfonylpyrimidyl-harnstoffe als Herbizide bekannt, welche im Pyrimidinteil durch Chlor substituiert sind. In den EP-A-84020 und 169815 werden Sulfonylharnstoffe beschrieben, die im Pyrimidinteil durch den Difluormethoxy- bzw. den Bromdifluormethoxyrest substituiert sind. Diese Verbindungen lassen jedoch wegen der unbefriedigenden Selektivität gegen Schadpflanzen zu wünschen übrig.

Der Erfindung lagen daher neue Verbindungen aus der Klasse der Sulfonylpyrimidyl-harnstoffe mit verbesserten herbiziden Eigenschaften als Aufgabe zugrunde. Entsprechend dieser Aufgabe wurden die eingangs definierten Sulfonylharnstoffe gefunden.

Ferner wurde gefunden, daß die Verbindungen der Formel I sowie deren Alkali- und Erdalkalimetallsalze eine gute Selektivität gegen Schadpflanzen in Kulturen wie Getreide und Mais haben.

Außerdem wurden chemisch eigenartige Verfahren zur Herstellung der Verbindungen I gefunden. Im Vergleich zu dem Stand der Technik lassen sich die Sulfonylharnstoffe I entgegen der Erwartung regioselektiv und in hoher Ausbeute und Reinheit herstellen, wenn man ausgeht von substituierten 2-Amino-4-fluoralkoxy-6-pyrimidinen der allgemeinen Formel IIIa

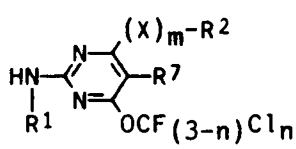

IIIa

in der m für 1 und n für 0 oder 1 stehen und die Substituenten folgende Bedeutung haben:

- $R^1$ Wasserstoff, $C_1$-$C_4$-Alkyl, $C_3$-$C_6$-Alkenyl oder $C_3$-$C_6$-Alkinyl;
- $R^2$ $C_1$-$C_4$-Alkyl, $C_3$-$C_6$-Alkenyl oder $C_3$-$C_6$-Alkinyl;
- $R^7$ Wasserstoff oder Halogen
- X O, S oder N-$R^4$, wobei
- $R^4$ für Wasserstoff oder $C_1$-$C_4$-Alkyl steht.

Diese Zwischenprodukte und deren Herstellung sind daher ebenfalls Gegenstand der Erfindung.

Zur Herstellung von im Pyrimidinteil halogensubstituierten Verbindungen ($R^2$ = Hal, m = 0) geht man von entsprechend substituierten 2-Amino-4-fluoralkoxy-6-halogen-pyrimidinen der Struktur IIIb aus (s. Formelschema 2), deren Herstellung Gegenstand der zeitgleichen deutschen Anmeldung P 40 07 316 (O.Z. 0050/41451) ist. Pyrimidinzwischenprodukte mit m = 0 und $R^2$ = Trifluormethyl erhält man in analoger Weise gemäß Formelschema 3.

Die erfindungsgemäßen Sulfonylharnstoffe der Formel I sind auf den in Formelschema 1 beschriebenen Wegen A, B und C zugänglich:

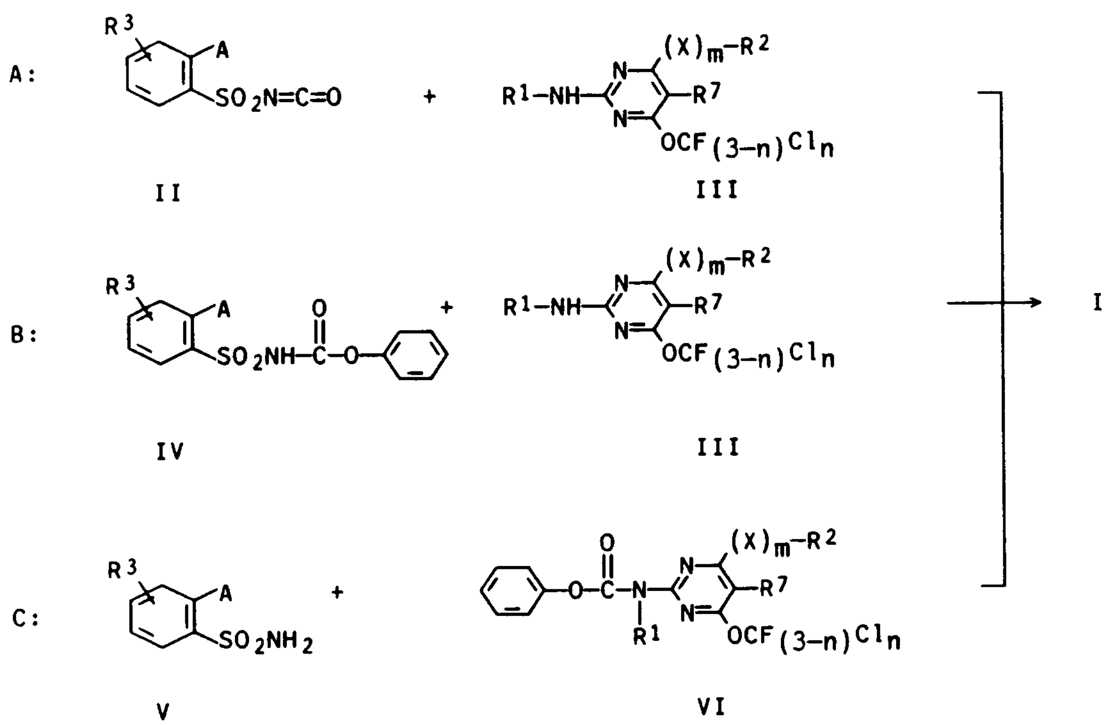
A:
II
III
B:
IV
III
C:
V
VI
I

Ausführungsform A

Man setzt ein Sulfonylisocyanat II in an sich bekannter Weise (EP-A-162 723) in einem inerten organischen Lösungsmittel mit ungefähr der stöchiometrischen Menge eines 2-Aminopyrimidinderivats III bei einer Temperatur von 0 bis 120 °C, vorzugsweise 10 bis 100 °C um. Die Reaktion kann drucklos oder unter Druck (bis 50 bar), vorzugsweise bei 1 bis 5 bar, kontinuierlich oder diskontinuierlich durchgeführt werden. Geeignete Lösungsmittel sind in der obengenannten Literatur aufgeführt.

Ausführungsform B:

Man setzt ein entsprechendes Sulfonylcarbamat der Formel IV in an sich bekannter Weise (EP-A-162 723) in einem inerten organischen Lösungsmittel bei einer Temperatur zwischen 0 und 120 °C, vorzugsweise 10 bis 100 °C mit einem 2-Aminopyrimidinderivat um. Es können hierbei Basen wie tertiäre Amine zugesetzt werden, wodurch die Reaktion beschleunigt und die Produktqualität verbessert werden.

Geeignete Basen hierfür sind z. B. tertiäre Amine wie Pyridin, die Picoline, 2,4- und 2,6-Lutidin, 2,4,6-Collidin, p-Dimethylaminopyridin, 1,4-Diaza(2,2,2)bicyclooctan (DABCO) und 1,8-Diazabicyclo(5,4,0)-undec-7-en.

Zweckmäßig verwendet man als Lösungsmittel die in der Literatur angegebenen und/oder Halogenkohlenwasserstoffe wie Dichlormethan und Chlorbenzol, Ether wie Diethylether, Tetrahydrofuran und Dioxan, Acetonitril, Dimethylformamid und/oder Essigsäureethylester in einer Menge von 100 bis 4000 Gew.%, vorzugsweise von 1000 bis 2000 Gew.%, bezogen auf die Ausgangsstoffe II, IV und V.

Im Rahmen der Herstellung der erfindungsgemäßen Verbindungen sind die 2-Aminopyrimidin-Zwischenprodukte III auf folgende vorteilhafte Weise zugänglich:

Formelschema 2

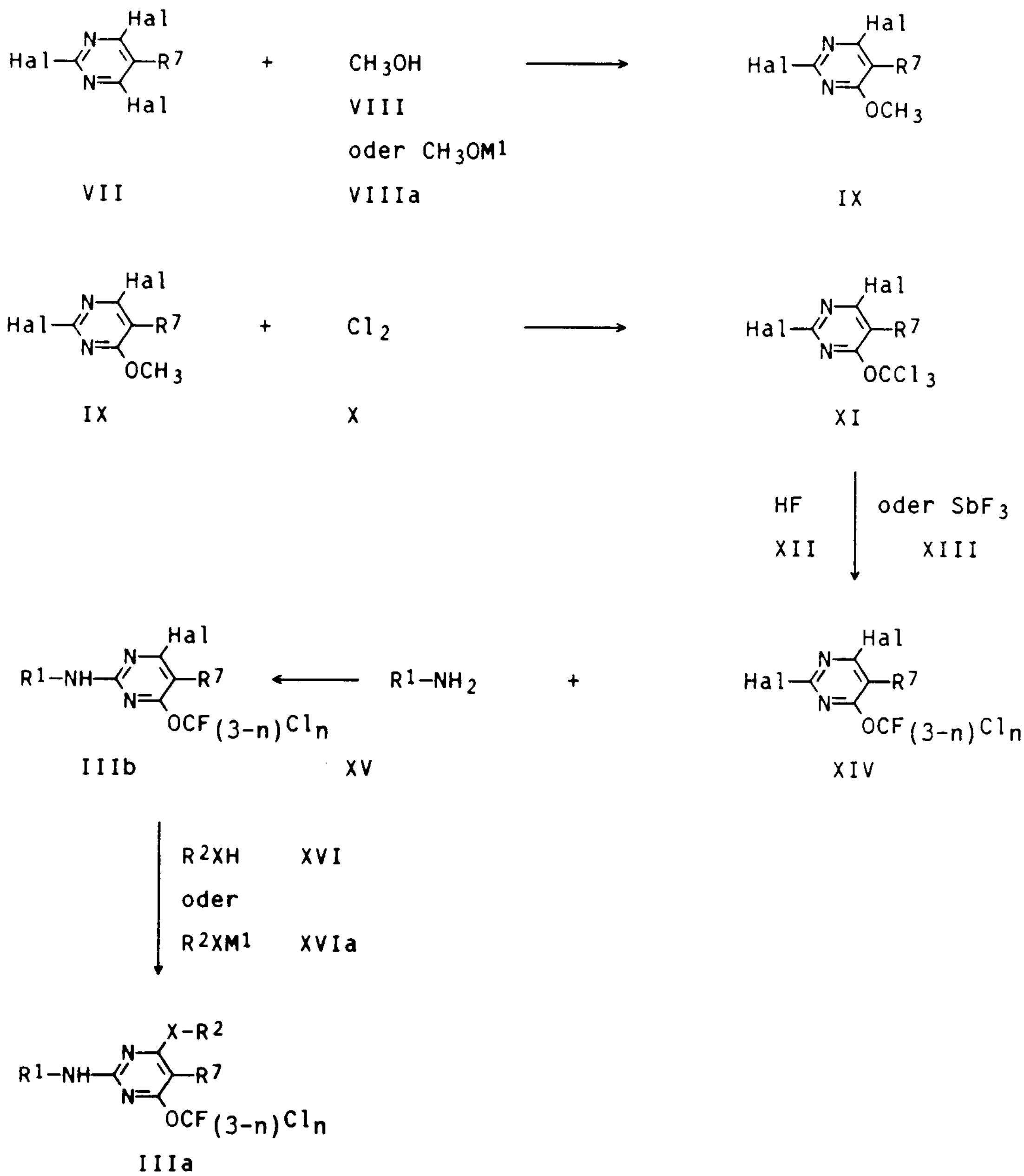


In entsprechender Weise gelangt man zu den 2-Amino-6-trifluormethylpyrimidinderivaten IIIc, wenn man anstelle der 2,4,6-Trihalogenverbindungen VII die entsprechenden 2,4-Dihalogen-6-trichlormethylpyrimidine gemäß Formelschema 3 umsetzt (s. Beispiele I.1, I.6 und I.12).

Formelschema 3

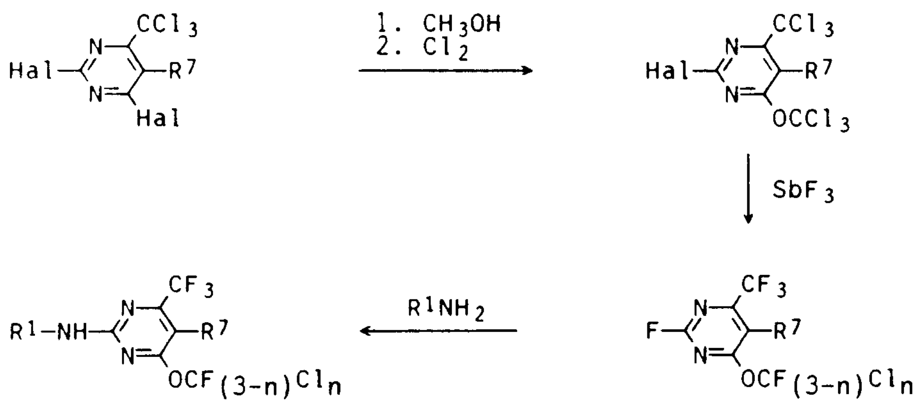


IIIc

Ausgehend von den Zwischenprodukten XIV in Formelschema 2 und Substitution des Halogenatoms in 4-Position durch die in Formelschema 3 beschriebene Reaktionssequenz (1. $CH_3OH$, 2. $Cl_2$, 3. $SbF_3$) sowie anschließende Umsetzung mit $R^1NH_2$ gelangt man zu den Zwischenprodukten IIId

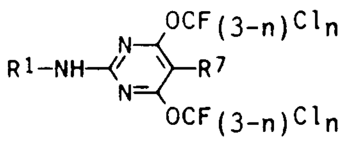

IIId

Entsprechend Formelschema 2 kann man beispielsweise ein 2,4,6-Trihalogenpyrimidin VII, bekannt aus J.Med.Chem. 6, 688 (1963) oder käuflich zu erwerben, in einem aprotisch polaren Lösungsmittel

a) mit Methanol VIII in Anwesenheit oder Abwesenheit einer Base oder

b) mit einem Methanolat VIIIa in Gegenwart von Methanol VIII bei Temperaturen zwischen -40 und 120 °C zum Methoxy-pyrimidin IX umsetzen. Diese Reaktionen können drucklos oder unter Druck (1 bis 10 bar, vorzugsweise 1 bis 5 bar), kontinuierlich oder diskontinuierlich durchgeführt werden.

Hal in Formel VII steht für Fluor, Chlor oder Brom.

$M^1$ in Formel VIIIa bedeutet ein Alkalimetallkation wie Lithium-, Natrium- und Kaliumkation oder das Equivalent eines Erdalkalimetallkations wie Magnesium-, Calcium- und Bariumkation.

Für die Umsetzung des Trihalogenpyrimidins mit Methanol VIII sind folgende Lösungsmittel geeignet: Ether wie Methyl-tert.-butylether, Diethylether, Ethylpropylether, n-Butylethylether, Di-n-butylether, Diisobutylether, Diisoamylether, Di-isoopropylether, Cyclohexylmethylether, Tetrahydrofuran, 1,2-Dimethoxyethan, Diethylenglycoldimethylether und Anisol, chlorierte Kohlenwasserstoffe wie 1,1,2,2-Tetrachlorethan, 1,1-Dichlorethylen, Chlorbenzol, 1,2-Dichlorbenzol und 1-Chlornaphthalin sowie entsprechende Gemische.

Zweckmäßig verwendet man das Lösungsmittel in einer Menge von 100 bis 2000 Gew.%, vorzugsweise 500 bis 1500 Gew.%, bezogen auf den Ausgangsstoff VII.

Zweckmäßig führt man jedoch die Umsetzung der Ausgangsstoffe VII mit VIII direkt in überschüssigem Methanol VIII als Lösungsmittel durch. Gegebenenfalls gibt man ein Alkalimethanolat VIIIa in einer equivalenten Menge oder einem über- oder Unterschuß von bis zu 5 %, bezogen auf den Ausgangsstoff VII, zu einer Suspension des Ausgangsstoffes VII in der 5-bis 20-fachen Gewichtsmenge Alkohol VIII als Lösungsmittel, bezogen auf den Ausgangsstoff VII, innerhalb bis zu einer Stunde bei einer Temperatur von -20 bis 80 °C. Zur Beendigug der Umsetzung rührt man dann noch 1/2 bis 8 Stunden bei 0 bis 120 °C, vorzugsweise 0 bis 100 °C nach.

Zur Isolierung der Methoxy-pyrimidine dienen die hierfür in der Literatur üblichen Aufarbeitungsmethoden wie destillative oder chromatographische Aufarbeitung.

Die Chlorierung des Methoxy-pyrimidins IX mit Chlor X zu dem Trichlormethoxy-pyrimidin XI führt man beispielsweise bei einer Temperatur von 60 bis 180 °C durch.

Als Chlorierungsmittel sind elementares Chlor oder Chlor abgebende Substanzen wie Sulfurylchlorid oder Phosphorpentachlorid geeignet. Chlor kann dabei auch in situ durch Oxydation von Chlorwasserstoffsäure, beispielsweise mit Braunstein oder durch anodische Chlorierung hergestellt werden.

Die Umsetzung kann in Gegenwart eines inerten Lösungsmittels, beispielsweise einem chlorierten Kohlenwasserstoff wie Chloroform, Tetrachlorkohlenstoff, Chlorbenzol, 1,2-, 1,3- oder 1,4-Dichlorbenzol, einem Nitril wie Acetonitril, Propionitril, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, $\alpha$-Chlorpropionsäurechlorid, $\alpha,\alpha$-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes IX durchgeführt werden.

Gegebenenfalls kann man die Reaktion durch Zugabe eines Radikalstarters beschleunigen; als solcher eignet sich die Bestrahlung mit Licht, vorzugsweise UV-Licht oder die Zugabe von $\alpha,\alpha'$-Azoisobutyronitril, zweckmäßig in einer Menge von 0,2 bis 7 mol%, bezogen auf den Ausgangsstoff IX. Man kann die Reaktion auch durch Zugabe eines Katalysators beschleunigen; als solcher eignet sich Phosphorpentachlorid, zweckmäßig in einer Menge von 0,5 bis 7 mol% bezogen auf den Ausgangsstoff IX. In diesem Fall legt man den Ausgangsstoff IX zusammen mit dem Katalysator vor und beginnt dann mit der Chlorierung. Statt des Phosphorpentachlorids kann man auch eine dieses unter den Reaktionsbedingungen bildende Ausgangskomponente, z.B. Phosphortrichlorid oder gelben Phosphor, zugeben und dann mit der Chlorierung beginnen.

Ausgangsstoff IX kann mit Chlor in annähernd stöchiometrischer Menge oder vorzugsweise im Überschuß, vorteilhaft mit 3,1 bis 11, insbesondere 3,3 bis 5 mol $Cl_2$ je Equivalent Methoxy in dem Ausgangsstoff IX umgesetzt werden. Die Umsetzung kann bei einer Temperatur von 60 bis 180°C, vorteilhaft von 100 bis 150°C, drucklos oder unter Druck kontinuierlich oder diskontinuierlich durchgefürt werden.

Chloriert man bei 1 bar, so werden zweckmäßig 3,3 bis 5 mol Chlorgas, bezogen auf ein Equivalent Methoxy in dem Ausgangsstoff IX, eingesetzt, was einem Chlorumsatz von 91 bis 60 % entspricht. Durch geeignete apparative Maßnahmen, z.B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, vorzugsweise 1 bis 10 Stunden die erforderliche Menge Chlorgas unter intensivem Rühren in den flüssigen Ausgangsstoff IX einleitet, wobei man zunächst bei einer Temperatur von 60 bis 80°C beginnt und diese - gegebenenfalls unter Ausnutzung des exothermen Charakters der Reaktion - kontinuierlich steigert, so daß gegen Ende die Umsetzung bei einer Temperatur von 100 bis 150°C durchgeführt wird. Bei größeren Reaktionsansätzen trägt man dem exothermen Charakter durch äußere Kühlung bzw. geeignete Dosierung der Chlormenge Rechnung; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Die Umsetzung des Trichlormethoxy-pyrimidins XI mit einem Halogenaustauschmittel führt man beispielsweise bei einer Temperatur von 0 bis 170°C durch.

Als Halogenaustauschmittel sind Antimontrifluorid in Gegenwart oder Abwesenheit katalytischer Mengen eines Antimon(V)salzes, z.B. Antimon(V)chlorid oder Fluorwasserstoff geeignet.

Zweckmäßig verwendet man einen Überschuß von 1 bis 200, vorzugsweise 5 bis 20 mol% Antimontrifluorid pro Trichlormethylequivalent. Die Katalysatormenge an Antimon(V)salz beträgt zwischen 1 bis 20, vorzugsweise 5 bis 18 mol% pro Trichlormethylequivalent. Vorzugsweise dosiert man den Ausgangsstoff XI bei 90 bis 130°C zu der Mischung des Halogenaustauschmittels und erwärmt dann noch zwischen 10 und ca. 120 Minuten auf eine Temperatur zwischen 140 bis 170°C. Anschließend wird durch Destillation aufgearbeitet.

Man kann die Reaktion jedoch auch kontinuierlich führen, den Ausgangsstoff XI bei 140 bis 170°C innerhalb 10 bis ca. 120 Minuten zugeben und gleichzeitig unter vermindertem Druck den entstehenden niedriger siedenden Endstoff XIV abdestillieren. Spuren mitgerissender Antimonsalze lassen sich durch Extraktion mit konz. Salzsäure beseitigen.

Arbeitet man ohne Antimon(V)salz-Katalyse oder setzt nur geringe Mengen, z. B. 0,2 bis 1 mol% ein, und reduziert die Menge an Antimontrifluorid auf 60 bis 90 mol% pro Trichlormethylequivalent, so bleibt der Halogenaustausch auf der Chlordifluormethoxystufe stehen.

Anstelle von Antimontrifluorid kann der Halogenaustausch auch mit Fluorwasserstoff bei 0 bis 170 °C, vorzugsweise 40 bis 120 °C, durchgeführt werden. Hierzu versetzt man in einem Autoklaven den Ausgangsstoff XI mit einem Überschuß von 300 bis 700, vorzugsweise 350 bis 400 mol% Fluorwasserstoff pro Trichlormethylequivalent und rührt 10 Minuten bis ca. 10 Stunden. Nach dem Entspannen und der Entfernung flüchtiger Bestandteile wird wie beschrieben aufgearbeitet.

Die Umsetzung des Fluormethoxy-pyrimidins XIV mit einem Amin XV führt man beispielsweise bei einer Temperatur von -80 bis 40 °C durch.

In Formel XV steht $R^1$ beispielsweise für Wasserstoff, $C_1$-$C_4$-Alkyl wie Methyl, Ethyl, n-Propyl, i-Propyl, n-Butyl, sek.-Butyl, i-Butyl, tert.-Butyl; $C_3$-$C_4$-Alkenyl wie 2-Propenyl, 2-Methylethenyl, 2-Butenyl, 3-Butenyl, 1-Methyl-2-propenyl oder 2-Methyl-2-propenyl; $C_3$-$C_4$-Alkinyl wie Propargyl, 2-Butinyl, 3-Butinyl oder 1-Methyl-2-propinyl.

Unter den Aminen, welche eingesetzt werden können, sollen die folgenden erwähnt werden: Ammoniak, Methylamin, Ethylamin, n-Propylamin, Isopropylamin, n-Butylamin, Isobutylamin, sek.-Butylamin, tert.-Butylamin, 2-Propenylamin, 2-Methylethenylamin, 2-Butenylamin, 3-Butenylamin, 1-Methyl-2-propenylamin, 2-Methyl-2-propenylamin, Propargylamin, 2-Butinylamin, 3-Butinylamin und 1-Methyl-2-propinylamin.

Die 2,6-Dihalogen-pyrimidine XIV können in einem aprotisch polaren Lösungsmittel mit den Aminen XV bei einer Temperatur von -80 bis 40 °C umgesetzt werden, wobei man das Amin XV entweder in einem Überschuß einsetzt oder eine organische Hilfsbase verwendet.

Für die Umsetzung des 2,6-Dihalogen-pyrimidins XIV mit dem Amin XV sind beispielsweise folgende Lösungsmittel geeignet:

Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid, 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf den Ausgangsstoff XIV.

Vorteilhaft gibt man 1,8 bis 2,5, insbesondere 1,95 bis 2,2 mol-Equivalent des Amins XV, bezogen auf den Ausgangsstoff XIV innerhalb 0,5 bis 2 Stunden zu einer Mischung von Ausgangsstoff XIV in einem der vorgenannten Lösungsmittel bei (-80) bis 40 °C, vorzugsweise -70 bis 25 °C, rührt bis zur Vervollständigung der Reaktion bis zu 3 Stunden nach und läßt dann zur Aufarbeitung auf 25 °C erwärmen.

Setzt man nur ungefähr stöchiometrische Mengen des Amins XV ein, so verwendet man zweckmäßig 0,9 bis 1,1 Equivalente einer organischen Hilfsbase, bezogen auf den Ausgangsstoff XIV. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, $\alpha$-,$\beta$-, $\gamma$-Picolin, 2,4- und 2,6-Lutidin und Triethylendiamin.

Die Reaktion kann drucklos oder unter Druck kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z. B. durch Chromatographie. Man kann jedoch auch direkt die organische Phase einengen und den Rückstand mit einem Lösungsmittel verrühren.

Die erfindungsgemäßen 2-Amino-4-fluoralkoxy-pyrimidine der Formel IIIa erhält man vorteilhaft in der Weise, daß man 2-Amino-4-fluoralkoxy-6-halogen-pyrimidine der Formel IIIb

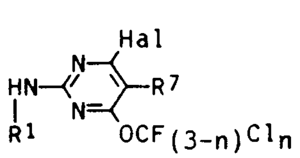

IIIb

in der Hal für Fluor, Chlor oder Brom steht und $R^1$ und n die vorgenannte Bedeutung haben, mit einem Nucleophil der Formel XVI,

$H$-$X$-$R^2$ XVI

in der X und $R^2$ die vorgenannte Bedeutung besitzen, oder dessen Salz umsetzt.

Die Umsetzung kann für den Fall der Verwendung von 2-Amino-4-fluor-6-trifluormethoxypyrimidin und Methylamin durch folgendes Schema beschrieben werden:

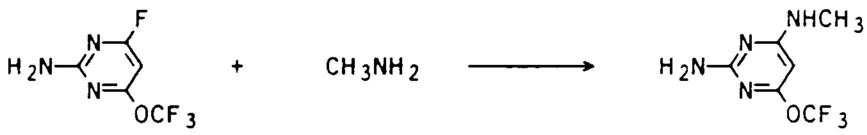

Für den Fall der Verwendung von 2-Amino-4-fluor-6-chlordifluormethoxy-pyrimidin und Natriummethylat läßt sich die Umsetzung durch folgendes Schema wiedergeben:

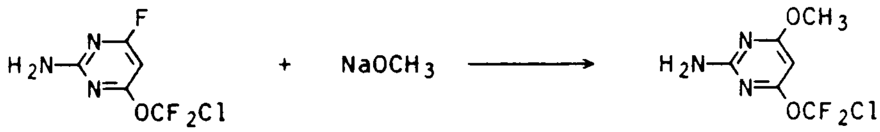

Das Verfahren liefert auf einfachem und wirtschaftlichem Weg neue 2-Amino-4-fluoralkoxy-pyrimidine in hoher Ausbeute und Reinheit. Entgegen der Erwartung werden Fluoralkoxygruppen nicht substituiert. Auch das in der Etherseitenkette stehende Chloratom bleibt trotz der alkalischen Reaktionsbedingungen erhalten. Im Hinblick auf den Stand der Technik (s. z.B. EP-A-70804) sind alle diese vorteilhaften Eigenschaften überraschend.

Bevorzugte Zwischenprodukte IIIa und dementsprechend bevorzugte Ausgangsstoffe IIIb sind solche, in deren Formeln $R^1$ und $R^2$ $C_1$-$C_4$-Alkyl wie Methyl, Ethyl, n-Propyl, i-Propyl, n-Butyl, sek.-Butyl, i-Butyl, tert.-Butyl; $C_3$-$C_4$-Alkenyl wie 2-Propenyl, 2-Methylethenyl, 2-Butenyl, 3-Butenyl, 1-Methyl-2-propenyl, 2-Methyl-2-propenyl; $C_3$-$C_4$-Alkinyl wie Propargyl, 2-Butinyl, 3-Butinyl, 1-Methyl-2-propinyl, darüberhinaus kann $R^1$ auch Wasserstoff bedeuten.

X O, S oder N-$R^4$, wobei

$R^4$ für Wasserstoff, Methyl, Ethyl, n-Propyl, i-Propyl, n-Butyl, sek.-Butyl, i-Butyl oder tert.-Butyl steht,

$R^7$ Wasserstoff und

n 0 oder 1

bedeuten.

Die Umsetzung des 2-Amino-4-fluoralkoxy-pyrimidins IIIb mit einem Nucleophil XVI oder dessen Salz XVIa führt man beispielsweise bei einer Temperatur von -80 bis 80 °C durch. Als Nucleophile XVI sind Ammoniak, aliphatische Amine, Alkohole und Thiole geeignet.

Unter den Aminen, welche als Nucleophile eingesetzt werden können, sollen die folgenden erwähnt werden: Ammoniak, Methylamin, Ethylamin, n-Propylamin, Isopropylamin, n-Butylamin, Isobutylamin, sek.-Butylamin, tert.-Butylamin, 2-Propenylamin, 2-Methylethenylamin, 2-Butenylamin, 3-Butenylamin, 1-Methyl-2-propenylamin, 2-Methyl-2-propenylamin, Propargylamin, 2-Butinylamin, 3-Butinylamin und 1-Methyl-2-propinylamin, Dimethylamin, Diethylamin, Di-n-propylamin, Di-n-butylamin, N-Methyl-ethylamin, N-Ethyl-n-propylamin, N-Methyl-allylamin und N-Methyl-propargylamin.

Unter den Alkoholen, welche als Nucleophile eingesetzt werden können, sollen die folgenden erwähnt werden: Methanol, Ethanol, n-Propanol, i-Propanol, n-Butanol, i-Butanol, sek.-Butanol, tert.-Butanol, 2-Propenol, 2-Methylethenol, 2-Butenol, 3-Butenol, 1-Methyl-2-propenol, 2-Methyl-2-propenol, Propinol, 2-Butinol, 3-Butinol und 1-Methyl-2-propinol.

Unter den Thiolen, welche als Nucleophile eingesetzt werden können, sollen die folgenden erwähnt werden: Methanthiol, Ethanthiol, n-Propanthiol, i-Propanthiol, n-Butanthiol, i-Butanthiol, sek.-Butanthiol, tert.-Butanthiol, 2-Butenthiol, 2-Methylethenthiol, 2-Butenthiol, 3-Butenthiol, 1-Methyl-2-propenthiol, 2-Methyl-2-propenthiol, Propinthiol, 2-Butinthiol, 3-Butinthiol und 1-Methyl-2-propinthiol.

Die 4-Halogen-pyrimidine IIIb können in einem aprotisch polaren Lösungsmittel mit den Aminen XVI bei einer Temperatur von -80 bis +80 °C, vorteilhaft -30 bis +20 °C umgesetzt werden, wobei man das Amin XVI entweder in einem Überschuß einsetzt oder eine organische Hilfsbase verwendet.

Für die Umsetzung des 4-Halogen-pyrimidins IIIb mit dem Amin XVI sind folgende Lösungsmittel geeignet:

Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf den Ausgangsstoff IIIb.

Vorteilhaft gibt man 1,8 bis 2,5, insbesondere 1,95 bis 2,2 mol-Equivalente des Amins XVI, bezogen auf den Ausgangsstoff IIIb innerhalb 0,5 bis 2 Stunden zu einer Mischung von Ausgangsstoff IIIb in einem der vorgenannten Lösungsmittel bei (-80) bis 80 °C, vorzugsweise -30 bis 25 °C, rührt bis zur Vervollständigung der Reaktion (ca. 3 Stunden) nach und läßt dann zur Aufarbeitung auf 25 °C erwärmen.

Setzt man nur ungefähr stöchiometrische Mengen des Amins XVI ein, so setzt man zweckmäßig 0,9 bis 1,1 Equivalente einer organischen Hilfsbase, bezogen auf den Ausgangsstoff IIIb, zu. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, α-,β-, γ-Picolin, 2,4- und 2,6-Lutidin und Triethylendiamin.

Wird die Umsetzung mit Alkoholen oder Thiolen durchgeführt, kann man analog der für Amine beschriebenen Reaktionsweise verfahren. Vorteilhaft gibt man das Nucleophil in einer Menge von 0,9 bis 1,3 mol-Equivalenten, bezogen auf den Ausgangsstoff IIIb innerhalb 0,5 bis 2 Stunden zusammen mit einer der vorgenannten Hilfsbasen zu einer Mischung von Ausgangsstoff IIIb in einem der vorgenannten Lösungsmittel bei -30 bis 20 °C, rührt bis zur Vervollständigung der Reaktion (ca. 3 Stunden) nach und läßt dann zur Aufarbeitung auf 25 °C erwärmen.

Neben den genannten sind als Lösungsmittel auch Ketone; z. B. Aceton, Methylethylketon; dipolare aprotische Lösungsmittel, z. B. Acetonitril, Dimethylformamid, Dimethylacetamid, Dimethylsulfoxid, N-Methylpyrrolidon, 1,3-Dimethylimidazolin-2-on; Aromaten, z. B. Benzol, Toluol, Xylol oder entsprechende Gemische geeignet. Vorteilhaft kann man im Fall des Einsatzes von Alkoholen als Nucleophile diese direkt als Lösungsmittel verwenden. Besonders bevorzugt sind Salze von Alkoholen oder Thiolen, die den Einsatz einer organischen Hilfsbase entbehrlich machen. Sie werden auf bekannte Weise unter Verwendung von Alkali- oder Erdalkalimetallen oder Metallhydriden, z. B. NaH, KH, $CaH_2$ oder LiH hergestellt.

Die Reaktion kann drucklos oder unter Druck, kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z.B. durch Chromatographie. Die Umsetzungsprodukte sind jedoch meist genügend rein, so daß man nur von dem ausgefallenen Salz abzufiltrieren und die organische Phase einzuengen braucht.

Bevorzugte Zwischenprodukte der Formel IIIa sind beispielsweise:
2-Amino-4-methoxy-6-trifluormethoxy-pyrimidin,
2-Amino-4-chlordifluormethoxy-6-methoxy-pyrimidin,
2-Amino-4-ethoxy-6-trifluormethoxy-pyrimidin,
2-Amino-4-chlordifluormethoxy-6-ethoxy-pyrimidin,
2-Amino-4-allyloxy-6-trifluormethoxy-pyrimidin,
2-Amino-4-allyloxy-6-chlordifluormethoxy-pyrimidin,
2-Amino-4-methylthio-6-trifluormethoxy-pyrimidin,
2-Amino-4-chlordifluormethoxy-6-methylthio-pyrimidin,
2-Amino-4-ethylthio-6-trifluormethoxy-pyrimidin,
2-Amino-4-chlordifluormethoxy-6-ethylthio-pyrimidin,
2-Amino-4-methylamino-6-trifluormethoxy-pyrimidin,
2-Amino-4-chlordifluormethoxy-6-methylamino-pyrimidin,
2-Amino-4-ethylamino-6-trifluormethoxy-pyrimidin,
2-Amino-4-chlordifluormethoxy-6-ethylamino-pyrimidin,
2-Amino-4-dimethylamino-6-trifluormethoxy-pyrimidin,
2-Amino-4-chlordifluormethoxy-6-dimethylamino-pyrimidin,
4-Methoxy-2-methylamino-6-trifluormethoxy-pyrimidin,
4-Chlordifluormethoxy-6-methoxy-2-methylamino-pyrimidin,
4-Ethoxy-2-methylamino-6-trifluormethoxy-pyrimidin,
4-Chlordifluormethoxy-6-ethoxy-2-methylamino-pyrimidin,
2,4-Bis-methylamino-6-trifluormethoxy-pyrimidin,
4-Chlordifluormethoxy-2,6-bis-methylamino-pyrimidin,
4-Ethylamino-2-methylamino-6-trifluormethoxy-pyrimidin,
4-Chlordifluormethoxy-6-ethylamino-2-methylamino-pyrimidin,
4-Dimethylamino-2-methylamino-6-trifluormethoxy-pyrimidin,
4-Chlordifluormethoxy-6-dimethylamino-2-methylamino-pyrimidin

Ausführungsform C:

Man setzt ein Sulfonamid der Formel V in an sich bekannter Weise (EP-A-141 777) in einem inerten organischen Lösungsmittel mit ungefähr der stöchiometrischen Menge eines Phenylcarbamats VI bei einer Temperatur von 0 bis 120 °C, vorzugsweise 20 bis 100 °C um. Die Reaktion kann drucklos oder unter Druck (bis 50 bar), vorzugsweise bei 1 bis 5 bar, kontinuierlich oder diskontinuierlich durchgeführt werden.

Geeignete Lösungsmittel sind neben den in der oben zitierten Literatur aufgeführten, z.B. Nitrokohlenwasserstoffe wie Nitroethan und Nitrobenzol, Nitrile wie Acetonitril und Benzonitril, Ester wie Essigsäureethylester, Amide wie Dimethylformamid und/oder Ketone wie Aceton. Bevorzugt wird die Umsetzung in Essigsäureethylester als Lösungsmittel und mit Pyridin oder einem der vorstehend genannten tertiären Amin als Base.

Die als Ausgangsstoffe der Formel V benötigten Sulfonamide lassen sich aus substituierten Anilinen, z.B. Anthranilestern oder -imiden, 2-Halogenanilinen oder 2-Halogenalkylanilinen durch Meerwein-Reaktion und anschließende Umsetzung mit Ammoniak herstellen.

Verbindungen der Formel I, in der $R^5$ Wasserstoff bedeutet, erhält man durch Hydrolyse von Estern der Formel I, in der $R^5$ einen $C_1$-$C_6$-Alkylrest bedeutet. Die Hydrolyse wird mit mindestens der doppelten Menge einer Base wie Natrium- oder Kaliumhydroxid, zweckmäßig in einem Lösungsmittelgemisch mit der 2- bis 8-fachen Menge Methanol und der 10- bis 40-fachen Menge Wasser, bezogen auf das Gewicht des entsprechenden Esters der Formel I, bei 30 bis 80 °C während 1 bis 20 Stunden durchgeführt. Durch Ansäuern fällt man die Sulfonamidcarbonsäuren der Formel I aus.

Im Hinblick auf die biologische Wirksamkeit sind Verbindungen der Formel I bevorzugt, in denen die Substituenten folgende Bedeutung haben:

- $R^1$ Wasserstoff und Methyl,
- $R^2$ Fluor, Chlor, Brom und Trifluormethyl, (m = 0), ferner Methyl, Ethyl, n-Propyl und Isopropyl (m = 1),
- $R^3$ Wasserstoff, Fluor, Chlor, Brom, Methyl, Methoxy und Trifluormethyl,
- X Sauerstoff, Schwefel und eine Aminogruppe -$NR^4$, wobei
- $R^4$ für Wasserstoff, Methyl und Ethyl steht,
- A Chlor, Trifluormethyl, eine Carboxylatgruppe und eine Carboxamidgruppe,
- $R^5$ eine $C_1$-$C_3$-Alkylgruppe wie Methyl, Ethyl, n-Propyl und Isopropyl,
  eine Alkenylgruppe wie Allyl, Crotyl und Buten-1-en-3-yl;
  eine Alkinylgruppe wie Propargyl, But-1-in-3-yl und But-2-inyl;
  Halogenalkyl wie 2-Chlorethyl, 2-Chlor-n-propyl, 3-Chlor-n-propyl, 1-Chlorbut-2-yl, 2-Chlor-isobutyl, 4-Chlor-n-butyl, Chlor-tert.-butyl, 3-chlor-prop-2-yl und 2, 2, 2-Trifluorethyl;
  Alkoxyalkyl wie 2-Methoxyethyl, 3-Ethoxyethyl, 3-Methoxy-n-propyl, 2-Methoxy-n-propyl, 3-Methoxy-n-butyl, 1-Methoxy-but-2-yl, Methoxy-tert.-butyl, 2-Methoxy-n-butyl und 4-Methoxy-n-butyl;
  Alkoxyalkoxyalkyl wie 2-Methoxy-ethoxy-methyl, 2-(Ethoxy)-ethoxy-methyl, 2-(Propoxy)-ethoxymethyl, 2-Methoxy-ethoxy-ethyl, 2-(Ethoxy)-ethoxy-ethyl und 2-(Methoxy-methoxy)-ethyl;
  Halogenalkoxyalkyl wie 2-($\beta$-Chlorethoxy)-ethyl, 3-($\beta$-Chlorethoxy)-n-propyl und 3-($\gamma$-Chlor-n-propoxy)-n-propyl;
  Cycloalkyl wie Cyclopentyl und Cyclohexyl,
- $R^6$ Wasserstoff;
  Alkyl wie Methyl, Ethyl, n-Propyl, Isopropyl und n-Butyl
  oder gemeinsam mit $R^5$ Tetramethylen, Pentamethylen, Hexamethylen, Ethylenoxyethylen und Ethylen-N-methylimino-ethylen,
- $R^7$ Wasserstoff und
- n 0 oder 1.

Als Salze der Verbindungen der Formel I kommen landwirtschaftlich brauchbare Salze, beispielsweise Alkalimetallsalze wie das Kalium-oder Natriumsalz, Erdalkalimetallsalze, wie das Calcium-, Magnesium-oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe

gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen alkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.003 werden in einer Mischung gelöst, die aus 80 Gewichtsteilen Xylol, 10 Gewichtsteilen des Anlagerungsproduktes von 8 bis 10 mol Ethylenoxid an 1 mol Ölsäure-N-monoethanolamid, 5 Gewichtsteilen Calciumsalz der Dodecylbenzolsulfonsäure und 5 Gewichtsteilen des Anlagerungsproduktes von 40 mol Ethylenoxid an 1 mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2.001 werden in einer Mischung gelöst, die aus 40 Gewichtsteilen Cyclohexanon, 30 Gewichtsteilen Isobutanol, 20 Gewichtsteilen des Anlagerungsproduktes von 7 mol Ethylenoxid an 1 mol Isooctylphenol und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 3.001 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 5.001 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer

Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 6.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 7.001 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.001 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 2, vorzugsweise 0,01 bis 1 kg/ha aktive Substanz (a.S.).

Die Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden eingige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Kulturenliste:

| Botanischer Name | Deutscher Name |
| --- | --- |
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffel |
| Juglans regia | Walnußbaum |
| Lens culinaris | Linse |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Musa spp. | Obst- und Mehlbanane |
| Micotiana tabacum (N. rustica) | Takak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Beispielen wiedergegeben Vorschriften wurden unter entsprechender Abwandlung der Ausgangsverbindungen zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellen und untersuchten Verbindungen eine gleichartige Wirkung erwarten.

I Herstellung der Vorprodukte

Beispiel I.1

2-Chlor-4-trichlormethoxy-6-trichlormethylpyrimidin

a) 2-Chlor-4-methoxy-6-trichlormethylpyrimidin

293,1 g (1,692 mol) 30 %ige Natriummethylatlösung wurden innerhalb 1 1/2 Stunden bei 0 bis 5 °C unter Rühren zu einer Lösung von 434 g (1,692 mol) 2,6-Dichlor-4-trichlormethylpyrimidin in 1 l 1,2-Dichlorethan gegeben. Es wurde 1 Stunde bei 0 bis 5 °C und 12 Stunden bei 25 °C gerührt. Das Reaktionsgemisch wurde mit Wasser und mit gesättigter Kochsalzlösung extrahiert. Nach dem Trocknen über Magnesiumsulfat und Einengen erhielt man 423 g (95 % d. Th.) der Titelverbindung als fast farbloses Öl vom $n_D^{23}$ = 1.5552. $^1$H-NMR ($CDCl_3$) (ppm) $OCH_3$ (s/3H) 4,1; CH (s/1H) 7,25.

b) 2-Chlor-4-trichlormethoxy-6-trichlormethylpyrimidin

In eine Mischung von 210 g (0,802 mol) a) und 260 mg (0,0016 mol) $\alpha,\alpha'$-Azoisobutyronitril wurden unter UV-Bestrahlung und gaschromatographischer Kontrolle des Reaktionsverlaufs bei einer Temperatur von zunächst 110 °C Chlor eingeleitet, wobei sich auch nach Entfernen der Heizbades eine Reaktionstemperatur von 140 °C einstellte. Nach dem Abklingen der Reaktion wurden während 5 1/2 Stunden bei 120 °C insgesamt 341 g (4,8 mol) Chlor eingeleitet. Zu dem erkaltenden Reaktionsgemisch rührte man ab 40 °C 70 ml n-Pentan zur Ausfällung hinzu. Der Niederschlag wurde abgesaugt, mit Petrolether gewaschen und getrocknet, wobei man 163 g (55 % d. Th.) der Titelverbindung vom Fp. 67-69 °C erhielt.

Das Filtrat (113,8 g) bestand nach dem Gaschromatogramm zu 83 % aus der Titelverbindung, 4 % 2-Chlor-4-dichlormethoxy-6-trichlormethylpyrimidin und 9 % 2,4-Dichlor-6-trichlormethylpyrimidin. Die Gesamtausbeute der Titelverbindung betrug 87,6 % d. Th.

Beispiel I.2

2,4-Difluor-6-trichlormethoxy-pyrimidin

a) 2,4-Difluor-6-methoxypyrimidin
(Herstellung nach dem Verfahren der älteren deutschen Patentanmeldung P 39 00 471 (O.Z. 0050/40474)

Zu einer Mischung aus 250 g (1,865 mol) 2,4,6-Trifluorpyrimidin in 1,4 l Methanol wurden bei -20 °C innerhalb von 45 Minuten 335,8 g (1,865 mol) 30 %iges Natriummethylat (in Methanol) gegeben und weitere 30 Minuten bei dieser Temperatur gerührt. Anschließend ließ man auf 25 °C erwärmen und engte die Reaktionsmischung auf ca. 1/5 ihres Volumens ein.

Das so erhaltene Gemisch wurde zwischen Diethylether und Wasser verteilt, wonach die organische Phase über Magnesiumsulfat getrocknet und eingeengt wurde. Nach Destillation (1,1 m Kolonne, 3 mm V-Füllkörper) erhielt man 141,6 g (52 % d. Th.) der Titelverbindung von Kp.: 144-145 °C.

Aus dem Destillationsrückstand erhielt man durch Destillation über einen Normag-Aufsatz 114,4 g (42 % d. Th.) an 4,6-Difluor-2-methoxy-pyrimidin vom Kp.: 157-161 °C.

b) 2,4-Difluor-6-trichlormethoxypyrimidin

210 g (2,96 mol) Chlor wurden unter UV-Bestrahlung und gaschromatographischer Kontrolle des Reaktionsverlaufs während 2 1/2 Stunden unter Rühren bei 130 °C in 123 g (0,843 mol) 2,4-Difluor-6-methoxy-pyrimidin eingeleitet. Das Reaktionsgemisch wurde über eine 10-cm-Vigreux-Kolonne im Vakuum destilliert, wobei man 190,2 g (90,5 % d. Th.) der Titelverbindung vom Sdp. 40-43 °C/0,2 mbar erhielt.

Beispiel I.3

2,4-Dichlor-6-trichlormethoxy-pyrimidin

Unter Rühren, UV-Bestrahlung und gaschromatographischer Kontrolle des Reaktionsverlaufs wurden 303 g (4,27 mol) Chlor während 1/2 Stunde bei 80 °C, 1 Stunde bei 100 °C, 3 Stunden bei 120 °C und 3

Stunden bei 150 °C in eine Mischung von 209 g (1,168 mol) 2,6-Dichlor-4-methoxy-pyrimidin und 2 g (0,012 mol) $\alpha,\alpha'$-Azoisobutyronitril eingeleitet. Anschließend wurde das Reaktionsgemisch im Vakuum über eine 50-cm-Kolonne mit 4 mm V2-A-Raschigringen destilliert. Man erhielt 241,3 g (73 % d. Th.) der Titelverbindung vom Sdp. 87-88 °C/0,4 mbar; Fp. 55-56 °C.

Beispiel I.4

2,4-Difluor-6-trifluormethoxy-pyrimidin

49,9 g (0,2 mol) 2,4-Difluor-6-trichlormethoxy-pyrimidin wurde bei 100 °C innerhalb 15 Minuten unter Rühren zu einer Mischung von 39,3 g (0,22 mol) Antimontrifluorid und 9,38 g (0,031 mol) Antimonpentachlorid gegeben.

Innerhalb 25 Minuten wurde die Badtemperatur von 100 auf 150 °C erhöht und 30 Minuten nachgerührt, wobei sich zwischen 120 bis 125 °C Rückfluß einstellte. Durch anschließende Destillation erhielt man 37,1 g (92,7 % d. Th.) der Titelverbindung vom Sdp. 125-127 °C und $n_D^{23}$ = 1.3787.

Beispiel I.5

6-Chlordifluormethoxy-2,4-difluor-pyrimidin

93 g (0,373 mol) 2,4-Difluor-6-trichlormethoxy-pyrimidin wurden bei 100 °C innerhalb 10 Minuten unter Rühren zu einer Mischung von 44,5 g (0,249 mol) Antimontrifluorid und 0,94 g (0,0031 mol) Antimonpentachlorid gegeben. Innerhalb 25 Minuten wurde die Badtemperatur von 100 auf 175 °C erhöht, wobei sich bei 145 °C Rückfluß einstellte. Nach 1 1/2 Stunden Rühren wurde das Reaktionsprodukt bei 146-150 °C abdestilliert. Das Destillat wurde in 200 ml Methylenchlorid gelöst, 2x mit 6 n Salzsäure extrahiert und über Magnesiumsulfat getrocknet. Nach dem Einengen im Vakuum erhielt man als Rückstand die Titelverbindung vom $n_D^{23}$ = 1.4142 in einer Ausbeute von 63,7 g = 78,8 % d.Th.

Beispiel I.6

2-Fluor-4-trifluormethoxy-6-trifluormethyl-pyrimidin

80 g (0,219 mol) 2-Chlor-4-trichlormethyl-6-trichlormethoxy-pyrimidin wurden innerhalb 5 Minuten unter Rühren bei 100 °C zu einer Mischung von 93,9 g (0,525 mol) Antimontrifluorid und 18,7 g (0,0627 mol) Antimonpentachlorid gegeben. Innerhalb 10 Minuten wurde die Badtemperatur auf 140 °C erhöht und 1 Stunde nachgerührt, wobei sich starker Rückfluß einstellte. Das Reaktionsprodukt wurde bei 135-140 °C, gegen Schluß bei 95 °C/50 mbar, überdestilliert. Das Destillat wurde in Methylenchlorid aufgenommen, mit 6 n Salzsäure extrahiert und über Magnesiumsulfat getrocknet. Nach dem Einengen im Vakuum erhielt man die Titelverbindung in einer Ausbeute von 35,9 g (65,5 % d. Th.).

Beispiel I.7

2,4-Dichlor-6-trifluormethoxy-pyrimidin

115 g (0,407 mol) 2,4-Dichlor-6-trichlormethoxy-pyrimidin wurden innerhalb 5 Minuten unter Rühren bei 100 °C zu einer Mischung von 80 g (0,447 mol) Antimontrifluorid und 18,77 g (0,0627 mol) Antimonpentachlorid gegeben, wobei sich die Reaktionstemperatur bis auf 140 °C erhöhte. Es wurde noch 45 Minuten bei 150 °C gerührt. Zur Destillation wurde ein Druck von 210 mbar eingestellt, wobei die Titelverbindung bei 128 °C überging; letzte flüchtige Bestandteile wurden bei 110 °C/22 mbar übergetrieben. Das Destillat wurde in Methylenchlorid gelöst, 3x mit 6 n Salzsäure extrahiert und über Magnesiumsulfat getrocknet. Nach dem Einengen im Vakuum erhielt man die Titelverbindung in einer Ausbeute von 80 g (84,4 % d. Th.) als farbloses Öl vom $n_D^{25}$ = 1,4604.

Beispiel I.8

2-Amino-4-chlordifluormethoxy-6-fluor-pyrimidin

9,8 g (0,578 mol) gasförmiges Ammoniak wurden innerhalb einer Stunde bei -75 bis -70 °C unter Rühren in eine Mischung von 62,5 g (0,289 mol) 2,4-Difluor-6-chlordifluormethoxy-pyrimidin in 300 ml Tetrahydrofuran eingegast. Es wurde eine Stunde bei -70 °C gerührt und dann auf Raumtemperatur erwärmt. Der ausgefallene Niederschlag wurde abgesaugt, zwischen Essigester und Wasser verteilt und die organische Phase über Magnesiumsulfat getrocknet. Das Reaktionsfiltrat wurde eingeengt, in der obigen Essigesterphase gelöst, über Kieselgel mit Petrolether:Ether = 5:1 chromatographiert und eingeengt. Man erhielt 46,5 g (75,3 % d. Th.) der Titelverbindung als farblose Kristalle vom Fp. 77-80 °C.

Beispiel I.9

2-Amino-4-fluor-6-trifluormethoxy-pyrimidin

8,7 g (0,51 mol) gasförmiges Ammoniak wurden innerhalb 1 Stunde bei - 75 bis 70 °C unter Rühren in eine Mischung von 51 g (0,255 mol) 2,4-Difluor-6-trifluormethoxy-pyrimidin in 200 ml Diethylether eingegast. Es wurde noch 1 1/2 Stunden bei -70 °C und 1 Stunde bei Raumtemperatur gerührt. Das Reaktionsgemisch wurde im Vakuum eingeengt, in Methylenchlorid aufgenommen und mit Wasser extrahiert. Nach dem Trocknen der organischen Phase, Einengen und Chromatographieren über Kieselgel mit Petrolether:Ether = 8:1 erhielt man 38,1 g (75,6 % d. Th.) der Titelverbindung als farblose Kristalle vom Fp. 86-89 °C.

Beispiel I.10

2-Amino-4-chlor-6-trifluormethoxy-pyrimidin

4,3 g (0,25 mol) gasförmiges Ammoniak wurden innerhalb 45 Minuten unter Rühren bei -50 bis -45 °C in eine Mischung von 23,3 g (0,1 mol) 2,4-Dichlor-6-trifluormethoxy-pyrimidin in 150 ml Methyl-tert.-butylether eingeleitet. Es wurde 30 Minuten bei -50 °C, 1 Stunde bei -30 °C und 1 Stunde bei 25 °C gerührt. Der ausgefallene Niederschlag wurde abgesaugt, mit Wasser gewaschen und getrocknet, wobei man 5,4 g (33,1 % d. Th.) 4-Amino-2,4-dichlorpyrimidin vom Fp. 270-272 als Nebenprodukt erhielt. Das Filtrat wurde mit Wasser gewaschen, getrocknet, teilweise im Vakuum eingeengt und mit petrolether:Ether = 5:1 frakioniert chromatographiert, wobei man in den ersten Fraktionen 3 g (12,8 % d. Th.) Ausgangsmaterial als farbloses Öl und im Nachlauf 9 g (42 % d. Th.) der Titelverbindung als farblose Kristalle vom Fp. 55-56 °C erhielt. Der Umsatz betrug 48,3 %.

Beispiel I.11

4-Chlordifluormethoxy-6-fluor-2-methylamino-pyrimidin

20,3 g (0,0938 mol) 4-Chlordifluormethoxy-2,6-difluor-pyrimidin wurden in 150 ml Tetrahydrofuran vorgelegt und bei -70 bis -60 °C innerhalb 30 Minuten unter Rühren mit 5,8 g (0,188 mol) gasförmigem Methylamin versetzt. Es wurde jeweils 1 Stunde bei -70 °C, 0 °C und 25 °C gerührt. Nach dem Einengen des Reaktionsgemisches im Vakuum wurde der Rückstand mit Wasser verrührt, 2x mit Essigester extrahiert und der Extrakt über Magnesiumsulfat getrocknet. Es wurde zum Teil im Vakuum eingeengt und dann über Kieselgel mit Ether/Petrolether 1:5 fraktioniert chromatographiert. Die ersten Fraktionen enthielten die Titelverbindung vom Fp. 57-61 °C in einer Ausbeute von 12,5 g (58,5 %).

Beispiel I.12

2-Amino-4-trifluormethoxy-6-trifluormethyl-pyrimidin

4,7 g (0,278 mol) gasförmiges Ammoniak wurden unter Rühren bei -75 bis -70 °C innerhalb 1 Stunde in eine Mischung von 38,0 g (0,147 mol) 2-Fluor(chlor)-4-trifluormethoxy-6-trifluormethyl-pyrimidin in 150 ml Diethylether eingegast. Es wurde jeweils 2 Stunden bei -75 und nach dem Erwärmen bei 25 °C gerührt. Nach dem Absaugen von dem ausgefallenen Niederschlag wurde die organische Phase mit Wasser extrahiert, getrocknet und teilweise eingeengt. Nach dem Chromatographieren mit Methyl-tert.--butylether

über Kieselgel erhielt man 20,4 g (56,1 % d. Th.) der Titelverbindung vom Fp. 47-49 °C.

II. Herstellung der Zwischenprodukte IIIa

Beispiel II.1

2-Amino-4-methoxy-6-trifluormethoxy-pyrimidin

2,7 g (0,015 mol) 30 %iges Natriummethylat wurden innerhalb 15 Minuten unter Rühren bei -5 bis 0 °C zu 2,95 g (0,015 mol) 2-Amino-4-fluor-6-trifluormethoxy-pyrimidin in 50 ml Methanol gegeben. Nach 1 Stunde Rühren bei 0 °C und Erwärmen auf 25 °C wurde das Reaktionsgemisch im Vakuum eingeengt, mit Wasser verrührt und 2x mit Methylenchlorid extrahiert. Nach dem Trocknen und Einengen im Vakuum erhielt man 3,1 g (98 % d. Th.) der Titelverbindung mit $n_D^{25}$ = 1,4770.

Beispiel II.2

2-Amino-4-chlordifluormethoxy-6-methoxy-pyrimidin

26,1 g (0,145 mol) 30 %iges Natriummethylat wurden innerhalb 15 Minuten unter Rühren bei -10 bis 0 °C zu 31,0 g (0,145 mol) 2-Amino-4-chlordifluormethoxy-6-fluorpyrimidin in 300 ml Methanol gegeben. Es wurde 30 Minuten bei 0 °C und 1 Stunde bei 25 °C gerührt. Das Reaktionsgemisch wurde im Vakuum eingeengt und wie oben aufgearbeitet. Man erhielt 31,6 g (96,6 % d. Th.) der Titelverbindung als farbloses Öl mit $n_D^{22}$ = 1,5039.

Beispiel II.3

4-Chlordifluormethoxy-2-methylamino-6-methoxy-pyrimidin

4,7 g (0,026 mol) 30 %iges Natriummethylat wurden innerhalb 10 Minuten unter Rühren bei 0 °C zu 6,0 g (0,0263 mol) 4-Chlordifluormethoxy-6-fluor-2-methylamino-pyrimidin in 100 ml Methanol gegeben. Es wurde jeweils 1 Stunde bei 0 °C und bei 25 °C gerührt. Nach üblicher Aufarbeitung erhielt man 6,3 g (100 % d. Th.) der Titelverbindung vom Fp. 49-53 °C.

Beispiel II.4

4-Chlordifluormethoxy-6-dimethylamino-2-amino-pyrimidin

1,9 g (0,0417 mol) gasförmiges Dimethylamin wurden innerhalb 10 Minuten unter Rühren bei 0 °C in eine Mischung von 8,9 g (0,0417 mol) 2-Amino-4-chlordifluormethoxy-6-fluor-pyrimidin in 100 ml Tetrahydrofuran eingeleitet. Es wurde 1 Stunde bei 0 °C und 2 Stunden bei 25 °C gerührt. Nach üblicher Aufarbeitung erhielt man 9,7 g (97,5 % d. Th.) der Titelverbindung vom Fp. 127-130 °C.

III. Herstellung der Sulfonylharnstoffverbindungen I

Beispiel III.1

2-(((4-Fluor-6-trifluormethoxy-1,3-pyrimidin-2-yl)aminocarbonyl)-aminosulfonyl)-benzoesäuremethylester

3,6 g (0,015 mol) 2-Isocyanatosulfonyl-benzoesäuremethylester in 15 ml 1,2-Dichlorethan wurden innerhalb 15 Minuten bei 25 °C unter Rühren zu einer Mischung von 2,95 g (0,015 mol) 2-Amino-4-fluor-6-trifluormethoxy-pyrimidin in 100 ml 1,2-Dichlorethan gegeben und 12 Stunden bei 25 °C gerührt. Die Reaktionslösung wurde im Vakuum eingeengt und der Rückstand mit Ether/Petrolether 1:1 verrührt. Nach dem Absaugen und Trocknen erhielt man 4,8 g (73,3 % d. Th.) der Titelverbindung vom Fp. 157-161 °C. (Wirkstoffbeispiel 1.001)

Beispiel III.2

2-(((4-Chlor-6-trifluormethoxy-1,3-pyrimidin-2-yl)aminocarbonyl)-aminosulfonyl)-benzoesäure-ethylester

2,55 g (0,01 mol) 2-Isocyanatosulfonyl-benzoesäure-ethylester in 10 ml Methylenchlorid wurden innerhalb 10 Minuten unter Rühren bei 25 ° C zu einer Mischung von 2,1 g (0,01 mol) 2-Amino-4-chlor-6-trifluormethoxy-pyrimidinin 100 ml Methylenchlorid gegeben. Es wurde 12 Stunden bei 25 ° C gerührt und von wenig Unlöslichem abgesaugt. Nach dem Einengen des Filtrats im Vakuum, Verrühren des Rückstandes mit Ether/Petrolether 1:1, Absaugen und Trocknen erhielt man 4,0 g (85,4 % d. Th.) der Titelverbindung vom Fp. 148-151 ° C.
(Wirkstoffbeispiel 3.003)

Beispiel III.3

2-(((4-Methoxy-6-trifluormethoxy-1,3-pyrimidin-2-yl)-aminocarbonyl)-aminosulfonyl)-benzoesäuremethylester

4,8 g (0,02 mol) 2-Isocyanatosulfonyl-benzoesäuremethylester in 10 ml Acetonitril wurden innerhalb 15 Minuten unter Rühren bei 25 ° C zu einer Mischung von 4,1 g (0,02 mol) 2-Amino-4-methoxy-6-trifluormethoxy-pyrimidin in 100 ml Acetonitril gegeben und 12 Stunden nachgerührt. Nach dem Abtrennen des ausgefallenen Niederschlages (2,4 g vom Fp. 141-143 ° C) wurde das Filtrat im Vakuum eingeengt und mit Ether/Petrolether verrührt, abgesaugt und getrocknet. Man erhielt weitere 4,3 g der Titelverbindung vom Fp. 141-143 ° C. Die Gesamtausbeute betrug 6,7 g (74,4 % d. Th.).
(Wirkstoffbeispiel 5.001)

Beispiel III.4

2-(((4-Methoxy-6-trifluormethoxy-1,3-pyrimidin-2-yl)-aminocarbonyl)-aminosulfonyl)-benzoesäuremethylester-natriumsalz

2,4 g (0,053 mol) 2-(((4-Methoxy-6-trifluormethoxy-1,3-pyrimidin-2-yl)-aminocarbonyl)aminosulfonyl)-benzoesäuremethylester (Wirkstoff-beispiel 5.001) wurden in 50 ml Methanol gelöst, bei 25 ° C mit 1,0 g (0,053 mol) 30%iger Natriummethylatlösung in Methanol versetzt und 10 Minuten gerührt. Nach dem Abdestillieren des Lösungsmittels bei vermindertem Druck erhielt man 2,5 g (100 % d. Th.) der Titelverbindung vom Fp. 175 ° C (Zersetzung)
(Wirkstoffbeispiel 5.019)
In entsprechender Weise wurde die in den nachfolgenden Tabellen dargestellten Sulfonylharnstoffderivate hergestellt.

Tabelle 1:

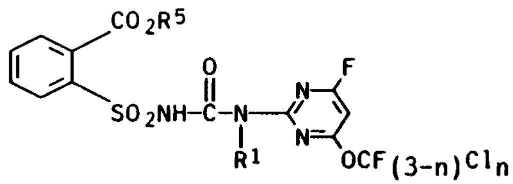

| Nr. | $R^1$ | $R^5$ | n | Fp ($^oC$) |
|---|---|---|---|---|
| 1.001 | H | $CH_3$ | 0 | 157-161 |
| 1.002 | $CH_3$ | $CH_3$ | 0 | |
| 1.003 | H | $CH_2CH_3$ | 0 | 148-150 |
| 1.004 | $CH_3$ | $CH_2CH_3$ | 0 | |
| 1.005 | H | $(CH_2)_2CH_3$ | 0 | |
| 1.006 | $CH_3$ | $(CH_2)_2CH_3$ | 0 | |
| 1.007 | H | $CH(CH_3)_2$ | 0 | 164-168 |
| 1.008 | H | $CH_2-CH=CH_2$ | 0 | |
| 1.009 | H | $CH_2-CH=CH-CH_3$ | 0 | |
| 1.010 | H | $CH_2-C\equiv C-CH_3$ | 0 | |
| 1.011 | H | $(CH_2)_2Cl$ | 0 | |
| 1.012 | $CH_3$ | $(CH_2)_2Cl$ | 0 | |

Tabelle 1: (Fortsetzung)

| Nr. | $R^1$ | $R^5$ | n | Fp ($^oC$) |  |
|---|---|---|---|---|---|
| 1.013 | H | $(CH_2)_2OCH_3$ | 0 |  |  |
| 1.014 | H | $(CH_2)_2O(CH_2)_2OCH_3$ | 0 |  |  |
| 1.015 | H | Cyclopentyl | 0 |  |  |
| 1.016 | H | Cyclohexyl | 0 |  |  |
| 1.017 | H | $CH_2CF_3$ | 0 |  |  |
| 1.018 | H | $(CH_2)_2SCH_3$ | 0 |  |  |
| 1.019 | H | $CH_3$ | 0 | 113 Zers. | Na-salz |
| 1.020 | $CH_3$ | $CH_3$ | 0 |  | Na-salz |
| 1.021 | H | $CH_2CH_3$ | 0 | 130 Zers. | Na-salz, |
| 1.022 | $CH_3$ | $CH_2CH_3$ | 0 |  | Na-salz |
| 1.023 | H | $(CH_2)_2CH_3$ | 0 |  | Na-salz |
| 1.024 | H | $(CH_2)_2Cl$ | 0 |  | Na-salz |
| 1.025 | H | $CH(CH_3)_2$ | 0 | 140-145 | Zers. Na-Salz |
| 1.026 | H | $C_2H_5$ | 0 | 135 Zers. | Ca-Salz |

Tabelle 2:

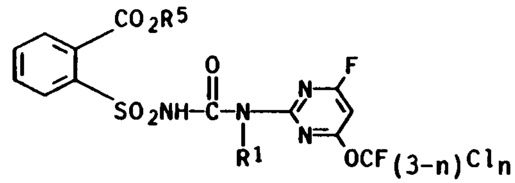

| Nr. | $R^1$ | $R^5$ | n | Fp (°C) |
|---|---|---|---|---|
| 2.001 | H | $CH_3$ | 1 | 167-170 |
| 2.002 | $CH_3$ | $CH_3$ | 1 | |
| 2.003 | H | $CH_2CH_3$ | 1 | |
| 2.004 | $CH_3$ | $CH_2CH_3$ | 1 | |
| 2.005 | H | $(CH_2)_2CH_3$ | 1 | |
| 2.006 | $CH_3$ | $(CH_2)_2CH_3$ | 1 | |
| 2.007 | H | $CH(CH_3)_2$ | 1 | |
| 2.008 | H | $CH_2-CH=CH_2$ | 1 | |
| 2.009 | H | $CH_2-CH=CH-CH_3$ | 1 | |
| 2.010 | H | $CH_2-C\equiv C-CH_3$ | 1 | |
| 2.011 | H | $(CH_2)_2Cl$ | 1 | |
| 2.012 | $CH_3$ | $(CH_2)_2Cl$ | 1 | |

Tabelle 2: (Fortsetzung)

| Nr. | $R^1$ | $R^5$ | n | Fp ($^oC$) | |
|---|---|---|---|---|---|
| 2.013 | H | $(CH_2)_2OCH_3$ | 1 | | |
| 2.014 | H | $(CH_2)_2O(CH_2)_2OCH_3$ | 1 | | |
| 2.015 | H | Cyclopentyl | 1 | | |
| 2.016 | H | Cyclohexyl | 1 | | |
| 2.017 | H | $CH_2CF_3$ | 1 | | |
| 2.018 | H | $(CH_2)_2SCH_3$ | 1 | | |
| 2.019 | H | $CH_3$ | 1 | 195 Zers. | Na-salz |
| 2.020 | $CH_3$ | $CH_3$ | 1 | | Na-salz |
| 2.021 | H | $CH_2CH_3$ | 1 | | Na-salz |
| 2.022 | $CH_3$ | $CH_2CH_3$ | 1 | | Na-salz |
| 2.023 | H | $(CH_2)_2CH_3$ | 1 | | Na-salz |
| 2.024 | H | $(CH_2)_2Cl$ | 1 | | Na-salz |

Tabelle 3:

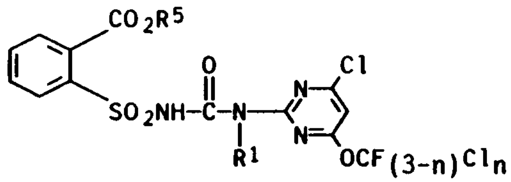

| Nr. | $R^1$ | $R^5$ | n | Fp ($^oC$) |
|---|---|---|---|---|
| 3.001 | H | $CH_3$ | 0 | 186-187 |
| 3.002 | $CH_3$ | $CH_3$ | 0 | |
| 3.003 | H | $CH_2CH_3$ | 0 | 148-151 |
| 3.004 | $CH_3$ | $CH_2CH_3$ | 0 | |
| 3.005 | H | $(CH_2)_2CH_3$ | 0 | |
| 3.006 | $CH_3$ | $(CH_2)_2CH_3$ | 0 | |
| 3.007 | H | $CH(CH_3)_2$ | 0 | |
| 3.008 | H | $CH_2-CH=CH_2$ | 0 | |
| 3.009 | H | $CH_2-CH=CH-CH_3$ | 0 | |
| 3.010 | H | $CH_2-C\equiv C-CH_3$ | 0 | |
| 3.011 | H | $(CH_2)_2Cl$ | 0 | |
| 3.012 | $CH_3$ | $(CH_2)_2Cl$ | 0 | |

Tabelle 3: (Fortsetzung)

| Nr. | $R^1$ | $R^5$ | n | Fp ($^oC$) | |
|---|---|---|---|---|---|
| 3.013 | H | $(CH_2)_2OCH_3$ | 0 | | |
| 3.014 | H | $(CH_2)_2O(CH_2)_2OCH_3$ | 0 | | |
| 3.015 | H | Cyclopentyl | 0 | | |
| 3.016 | H | Cyclohexyl | 0 | | |
| 3.017 | H | $CH_2CF_3$ | 0 | | |
| 3.018 | H | $(CH_2)_2SCH_3$ | 0 | | |
| 3.019 | H | $CH_3$ | 0 | 188 Zers. | Na-salz |
| 3.020 | $CH_3$ | $CH_3$ | 0 | | Na-salz |
| 3.021 | H | $CH_2CH_3$ | 0 | | Na-salz |
| 3.022 | $CH_3$ | $CH_2CH_3$ | 0 | | Na-salz |
| 3.023 | H | $(CH_2)_2CH_3$ | 0 | | Na-salz |
| 3.024 | H | $(CH_2)_2Cl$ | 0 | | Na-salz |

Tabelle 4:

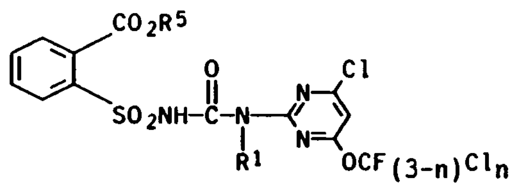

| Nr. | $R^1$ | $R^5$ | n | Fp (°C) |
|---|---|---|---|---|
| 4.001 | H | $CH_3$ | 1 | |
| 4.002 | $CH_3$ | $CH_3$ | 1 | |
| 4.003 | H | $CH_2CH_3$ | 1 | |
| 4.004 | $CH_3$ | $CH_2CH_3$ | 1 | |
| 4.005 | H | $(CH_2)_2CH_3$ | 1 | |
| 4.006 | $CH_3$ | $(CH_2)_2CH_3$ | 1 | |
| 4.007 | H | $CH(CH_3)_2$ | 1 | |
| 4.008 | H | $CH_2-CH=CH_2$ | 1 | |
| 4.009 | H | $CH_2-CH=CH-CH_3$ | 1 | |
| 4.010 | H | $CH_2-C\equiv C-CH_3$ | 1 | |
| 4.011 | H | $(CH_2)_2Cl$ | 1 | |
| 4.012 | $CH_3$ | $(CH_2)_2Cl$ | 1 | |

Tabelle 4: (Fortsetzung)

| Nr. | $R^1$ | $R^5$ | n | Fp (°C) |
|---|---|---|---|---|
| 4.013 | H | $(CH_2)_2OCH_3$ | 1 | |
| 4.014 | H | $(CH_2)_2O(CH_2)_2OCH_3$ | 1 | |
| 4.015 | H | Cyclopentyl | 1 | |
| 4.016 | H | Cyclohexyl | 1 | |
| 4.017 | H | $CH_2CF_3$ | 1 | |
| 4.018 | H | $(CH_2)_2SCH_3$ | 1 | |
| 4.019 | H | $CH_3$ | 1 | Na-salz |
| 4.020 | $CH_3$ | $CH_3$ | 1 | Na-salz |
| 4.021 | H | $CH_2CH_3$ | 1 | Na-salz |
| 4.022 | $CH_3$ | $CH_2CH_3$ | 1 | Na-salz |
| 4.023 | H | $(CH_2)_2CH_3$ | 1 | Na-salz |
| 4.024 | H | $(CH_2)_2Cl$ | 1 | Na-salz |

Tabelle 5:

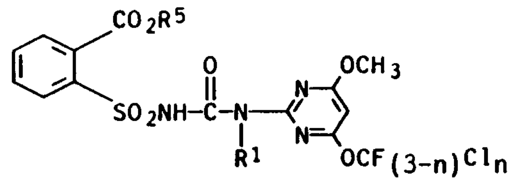

| Nr. | $R^1$ | $R^5$ | n | Fp (°C) |
|---|---|---|---|---|
| 5.001 | H | $CH_3$ | 0 | 141-143 |
| 5.002 | $CH_3$ | $CH_3$ | 0 | 95- 98 |
| 5.003 | H | $CH_2CH_3$ | 0 | 139-140 |
| 5.004 | $CH_3$ | $CH_2CH_3$ | 0 | |
| 5.005 | H | $(CH_2)_2CH_3$ | 0 | |
| 5.006 | $CH_3$ | $(CH_2)_2CH_3$ | 0 | |
| 5.007 | H | $CH(CH_3)_2$ | 0 | |
| 5.008 | H | $CH_2-CH=CH_2$ | 0 | |
| 5.009 | H | $CH_2-CH=CH-CH_3$ | 0 | |
| 5.010 | H | $CH_2-C\equiv C-CH_3$ | 0 | |
| 5.011 | H | $(CH_2)_2Cl$ | 0 | |
| 5.012 | $CH_3$ | $(CH_2)_2Cl$ | 0 | |

Tabelle 5: (Fortsetzung)

| Nr. | $R^1$ | $R^5$ | n | Fp ($^oC$) | |
|---|---|---|---|---|---|
| 5.013 | H | $(CH_2)_2OCH_3$ | 0 | | |
| 5.014 | H | $(CH_2)_2O(CH_2)_2OCH_3$ | 0 | | |
| 5.015 | H | Cyclopentyl | 0 | | |
| 5.016 | H | Cyclohexyl | 0 | | |
| 5.017 | H | $CH_2CF_3$ | 0 | | |
| 5.018 | H | $(CH_2)_2SCH_3$ | 0 | | |
| 5.019 | H | $CH_3$ | 0 | 175 Zers. | Na-salz, |
| 5.020 | $CH_3$ | $CH_3$ | 0 | 130 Zers. | Na-salz |
| 5.021 | H | $CH_2CH_3$ | 0 | 168 Zers. | Na-salz, |
| 5.022 | $CH_3$ | $CH_2CH_3$ | 0 | | Na-salz |
| 5.023 | H | $(CH_2)_2CH_3$ | 0 | | Na-salz |
| 5.024 | H | $(CH_2)_2Cl$ | 0 | | Na-salz |
| 5.025 | $CH_2-CH=CH_2$ | | 0 | 97-99 | |

Tabelle 6:

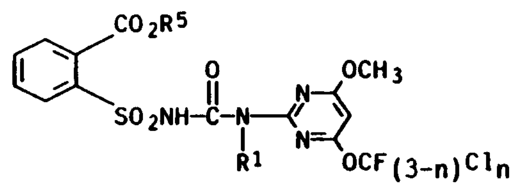

| Nr. | $R^1$ | $R^5$ | n | Fp (°C) |
|---|---|---|---|---|
| 6.001 | H | $CH_3$ | 1 | 124-127 |
| 6.002 | $CH_3$ | $CH_3$ | 1 | 79- 83 |
| 6.003 | H | $CH_2CH_3$ | 1 | 133-136 |
| 6.004 | $CH_3$ | $CH_2CH_3$ | 1 | |
| 6.005 | H | $(CH_2)_2CH_3$ | 1 | |
| 6.006 | $CH_3$ | $(CH_2)_2CH_3$ | 1 | |
| 6.007 | H | $CH(CH_3)_2$ | 1 | |
| 6.008 | H | $CH_2-CH=CH_2$ | 1 | |
| 6.009 | H | $CH_2-CH=CH-CH_3$ | 1 | |
| 6.010 | H | $CH_2-C\equiv C-CH_3$ | 1 | |
| 6.011 | H | $(CH_2)_2Cl$ | 1 | |
| 6.012 | $CH_3$ | $(CH_2)_2Cl$ | 1 | |

Tabelle 6: (Fortsetzung)

| Nr. | $R^1$ | $R^5$ | n | Fp (°C) | |
|---|---|---|---|---|---|
| 6.013 | H | $(CH_2)_2OCH_3$ | 1 | | |
| 6.014 | H | $(CH_2)_2O(CH_2)_2OCH_3$ | 1 | | |
| 6.015 | H | Cyclopentyl | 1 | | |
| 6.016 | H | Cyclohexyl | 1 | | |
| 6.017 | H | $CH_2CF_3$ | 1 | | |
| 6.018 | H | $(CH_2)_2SCH_3$ | 1 | | |
| 6.019 | H | $CH_3$ | 1 | 148 Zers. | Na-salz |
| 6.020 | $CH_3$ | $CH_3$ | 1 | | Na-salz |
| 6.021 | H | $CH_2CH_3$ | 1 | | Na-salz |
| 6.022 | $CH_3$ | $CH_2CH_3$ | 1 | | Na-salz |
| 6.023 | H | $(CH_2)_2CH_3$ | 1 | | Na-salz |
| 6.024 | H | $(CH_2)_2Cl$ | 1 | | Na-salz |

Tabelle 7:

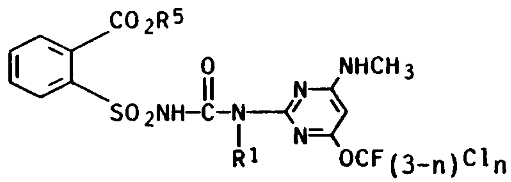

| Nr. | $R^1$ | $R^5$ | n | Fp (°C) |
|---|---|---|---|---|
| 7.001 | H | $CH_3$ | 0 | 188 Zers. |
| 7.002 | $CH_3$ | $CH_3$ | 0 | |
| 7.003 | H | $CH_2CH_3$ | 0 | 158 Zers. |
| 7.004 | $CH_3$ | $CH_2CH_3$ | 0 | |
| 7.005 | H | $(CH_2)_2CH_3$ | 0 | |
| 7.006 | $CH_3$ | $(CH_2)_2CH_3$ | 0 | |
| 7.007 | H | $CH(CH_3)_2$ | 0 | |
| 7.008 | H | $CH_2-CH=CH_2$ | 0 | |
| 7.009 | H | $CH_2-CH=CH-CH_3$ | 0 | |
| 7.010 | H | $CH_2-C\equiv C-CH_3$ | 0 | |
| 7.011 | H | $(CH_2)_2Cl$ | 0 | |
| 7.012 | $CH_3$ | $(CH_2)_2Cl$ | 0 | |

Tabelle 7: (Fortsetzung)

| Nr. | $R^1$ | $R^5$ | n | Fp (°C) | |
|---|---|---|---|---|---|
| 7.013 | H | $(CH_2)_2OCH_3$ | 0 | | |
| 7.014 | H | $(CH_2)_2O(CH_2)_2OCH_3$ | 0 | | |
| 7.015 | H | Cyclopentyl | 0 | | |
| 7.016 | H | Cyclohexyl | 0 | | |
| 7.017 | H | $CH_2CF_3$ | 0 | | |
| 7.018 | H | $(CH_2)_2SCH_3$ | 0 | | |
| 7.019 | H | $CH_3$ | 0 | 157 Zers. | Na-salz |
| 7.020 | $CH_3$ | $CH_3$ | 0 | | Na-salz |
| 7.021 | H | $CH_2CH_3$ | 0 | | Na-salz |
| 7.022 | $CH_3$ | $CH_2CH_3$ | 0 | | Na-salz |
| 7.023 | H | $(CH_2)_2CH_3$ | 0 | | Na-salz |
| 7.024 | H | $(CH_2)_2Cl$ | 0 | | Na-salz |
| 7.025 | $CH_2-CH=CH_2$ | $CH_3$ | 0 | 152-154 | |

Tabelle 8:

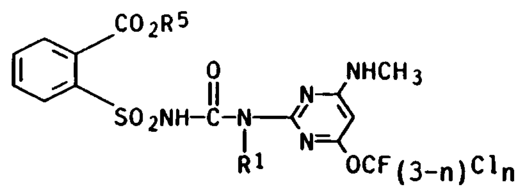

| Nr. | $R^1$ | $R^5$ | n | Fp ($^oC$) |
|---|---|---|---|---|
| 8.001 | H | $CH_3$ | 1 | 186 Zers. |
| 8.002 | $CH_3$ | $CH_3$ | 1 | |
| 8.003 | H | $CH_2CH_3$ | 1 | |
| 8.004 | $CH_3$ | $CH_2CH_3$ | 1 | |
| 8.005 | H | $(CH_2)_2CH_3$ | 1 | |
| 8.006 | $CH_3$ | $(CH_2)_2CH_3$ | 1 | |
| 8.007 | H | $CH(CH_3)_2$ | 1 | |
| 8.008 | H | $CH_2-CH=CH_2$ | 1 | |
| 8.009 | H | $CH_2-CH=CH-CH_3$ | 1 | |
| 8.010 | H | $CH_2-C\equiv C-CH_3$ | 1 | |
| 8.011 | H | $(CH_2)_2Cl$ | 1 | |
| 8.012 | $CH_3$ | $(CH_2)_2Cl$ | 1 | |

Tabelle 8: (Fortsetzung)

| Nr. | $R^1$ | $R^5$ | n | Fp (°C) | |
|---|---|---|---|---|---|
| 8.013 | H | $(CH_2)_2OCH_3$ | 1 | | |
| 8.014 | H | $(CH_2)_2O(CH_2)_2OCH_3$ | 1 | | |
| 8.015 | H | Cyclopentyl | 1 | | |
| 8.016 | H | Cyclohexyl | 1 | | |
| 8.017 | H | $CH_2CF_3$ | 1 | | |
| 8.018 | H | $(CH_2)_2SCH_3$ | 1 | | |
| 8.019 | H | $CH_3$ | 1 | 163 Zers. | Na-salz |
| 8.020 | $CH_3$ | $CH_3$ | 1 | | Na-salz |
| 8.021 | H | $CH_2CH_3$ | 1 | | Na-salz |
| 8.022 | $CH_3$ | $CH_2CH_3$ | 1 | | Na-salz |
| 8.023 | H | $(CH_2)_2CH_3$ | 1 | | Na-salz |
| 8.024 | H | $(CH_2)_2Cl$ | 1 | | Na-salz |

Tabelle 9:

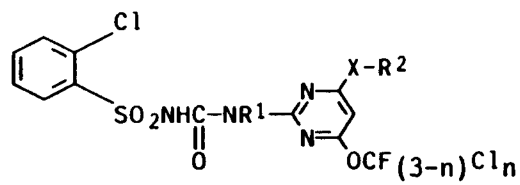

| Nr. | $R^1$ | X | $R^2$ | n | Fp (°C) | |
|---|---|---|---|---|---|---|
| 9.001 | H | - | F | 0 | 139-141 | |
| 9.002 | H | - | Cl | 0 | 181-183 | |
| 9.003 | $CH_3$ | - | F | 0 | | |
| 9.004 | $CH_3$ | - | Cl | 0 | | |
| 9.005 | H | - | F | 1 | | |
| 9.006 | H | - | Cl | 1 | | |
| 9.007 | $CH_3$ | - | F | 1 | | |
| 9.008 | $CH_3$ | - | Cl | 1 | | |
| 9.009 | H | - | F | 0 | | Na-Salz; |
| 9.010 | H | - | Cl | 0 | | Na-Salz; |
| 9.011 | H | O | $CH_3$ | 0 | 150-152 | |
| 9.012 | H | O | $CH_3$ | 1 | 142-143 | |
| 9.013 | $CH_3$ | O | $CH_3$ | 0 | | |
| 9.014 | $CH_3$ | O | $CH_3$ | 1 | | |

Tabelle 9: (Fortsetzung)

| Nr. | $R^1$ | X | $R^2$ | n | Fp ($^oC$) | |
|---|---|---|---|---|---|---|
| 9.015 | $CH_3$ | O | $CH_3$ | 0 | | Na-Salz |
| 9.016 | $CH_3$ | O | $CH_3$ | 1 | | Na-Salz |
| 9.017 | H | NH | $CH_3$ | 0 | 177-178 | |
| 9.018 | H | NH | $CH_3$ | 1 | 183-185 | |
| 9.019 | H | $NCH_3$ | $CH_3$ | 0 | 185-188 | |
| 9.020 | H | $NCH_3$ | $CH_3$ | 1 | | |
| 9.021 | $CH_3$ | NH | $CH_3$ | 0 | | |
| 9.022 | H | O | $CH_3$ | 0 | 180 Zers. | Na-Salz |
| 9.023 | H | O | $CH_3$ | 1 | 184 | Na-Salz |

Tabelle 10:

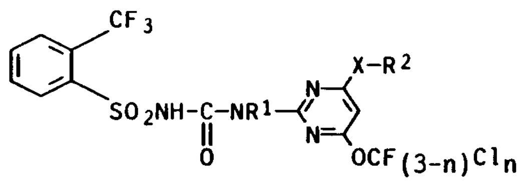

| Nr. | $R^1$ | X | $R^2$ | n | Fp (°C) | |
|---|---|---|---|---|---|---|
| 10.001 | H | - | F | 0 | | |
| 10.002 | H | - | Cl | 0 | | |
| 10.003 | H | - | F | 1 | | |
| 10.004 | H | - | Cl | 1 | | |
| 10.005 | H | O | $CH_3$ | 0 | | |
| 10.006 | H | O | $CH_3$ | 1 | | |
| 10.007 | H | NH | $CH_3$ | 0 | | |
| 10.008 | H | NH | $CH_3$ | 1 | | |
| 10.009 | $CH_3$ | NH | $CH_3$ | 0 | | |
| 10.010 | $CH_3$ | NH | $CH_3$ | 1 | | |
| 10.011 | H | O | $CH_3$ | 0 | | Na-Salz |
| 10.012 | H | O | $CH_3$ | 1 | 159 | Na-Salz |
| 10.013 | H | O | $CH_3$ | 1 | 150-153 | |
| 10.014 | H | O | $CH_3$ | 0 | 168-175 | |
| 10.015 | H | O | $CH_3$ | 0 | 146 | Na-Salz |
| 10.016 | H | NH | $CH_3$ | 1 | 192 | |

Tabelle 11:

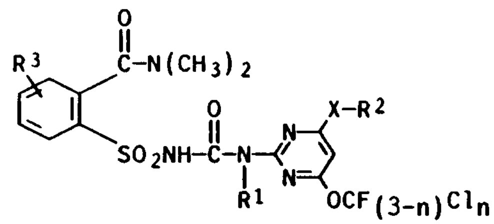

| Nr. | $R^1$ | X | $R^2$ | $R^3$ | n | Fp (°C) |
|---|---|---|---|---|---|---|
| 11.001 | H | - | F | - | 0 | |
| 11.002 | H | - | Cl | - | 0 | |
| 11.003 | H | - | F | - | 1 | |
| 11.004 | H | - | Cl | - | 1 | |
| 11.005 | H | O | $CH_3$ | - | 0 | |
| 11.006 | H | O | $CH_3$ | 3-F | 1 | |
| 11.007 | H | NH | $CH_3$ | - | 0 | |
| 11.008 | H | NH | $CH_3$ | - | 1 | |
| 11.009 | H | O | $CH_3$ | 5-Cl | 0 | |
| 11.010 | H | O | $CH_3$ | 5-Cl | 1 | |

Tabelle 12:

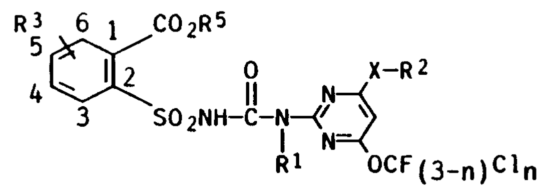

| Nr. | $R^1$ | X | $R^2$ | $R^3$ | $R^5$ | n | Fp (°C) |
|---|---|---|---|---|---|---|---|
| 12.001 | H | - | F | 3-F | $CH_3$ | 0 | |
| 12.002 | H | - | Cl | 3-F | $CH_3$ | 0 | |
| 12.003 | H | - | F | 3-F | $CH_3$ | 1 | |
| 12.004 | H | - | Cl | 3-F | $CH_3$ | 1 | |
| 12.005 | H | O | $CH_3$ | 3-F | $CH_3$ | 0 | 96-98 |
| 12.006 | H | O | $CH_3$ | 3-F | $CH_3$ | 1 | 85-86 |
| 12.007 | H | O | $CH_3$ | 5-Cl | $CH_3$ | 0 | |
| 12.008 | H | O | $CH_3$ | 5-Cl | $CH_3$ | 1 | |
| 12.009 | H | - | F | 5-Cl | $CH_3$ | 0 | |
| 12.010 | H | - | F | 5-Cl | $CH_3$ | 1 | |
| 12.011 | H | - | Cl | 5-Cl | $CH_3$ | 0 | |
| 12.012 | H | - | Cl | 5-Cl | $CH_3$ | 1 | |
| 12.013 | H | - | F | 6-$CH_3$ | $CH_3$ | 0 | |
| 12.014 | H | - | F | 6-$CH_3$ | $CH_3$ | 1 | |
| 12.015 | H | - | Cl | 6-$CH_3$ | $CH_3$ | 0 | |
| 12.016 | H | - | Cl | 6-$CH_3$ | $CH_3$ | 1 | |
| 12.017 | H | O | $CH_3$ | 6-$CH_3$ | $CH_3$ | 0 | |
| 12.018 | H | O | $CH_3$ | 6-$CH_3$ | $CH_3$ | 1 | |
| 12.019 | H | NH | $CH_3$ | 6-$CH_3$ | $CH_3$ | 0 | |

Tabelle 12: (Fortsetzung)

| Nr. | $R^1$ | X | $R^2$ | $R^3$ | $R^5$ | n | Fp (°C) | |
|---|---|---|---|---|---|---|---|---|
| 12.020 | H | NH | $CH_3$ | 6-$CH_3$ | $CH_3$ | 1 | | |
| 12.021 | $CH_3$ | NH | $CH_3$ | 6-$CH_3$ | $CH_3$ | 0 | | |
| 12.022 | H | NH | $CH_3$ | 6-$CH_3$ | $CH_3$ | 0 | | Na-Salz |
| 12.023 | H | - | $CF_3$ | - | $CH_3$ | 0 | 135-137 Zers. | |
| 12.024 | H | - | $CF_3$ | - | $CH_3$ | 0 | 137 Zers. | Na-Salz |
| 12.025 | $CH_3$ | - | $CF_3$ | - | $CH_3$ | 0 | | |
| 12.026 | H | - | $CF_3$ | - | $CH_3$ | 1 | | |
| 12.027 | H | N-$CH_3$ | $CH_3$ | - | $CH_3$ | 0 | 198 Zers. | |
| 12.028 | H | N-$CH_3$ | $CH_3$ | - | $CH_3$ | 0 | 160 Zers. | Na-Salz |
| 12.029 | H | N-$CH_3$ | $CH_3$ | 3-$CH_3$ | $CH_3$ | 0 | 160-165 Zers. | |
| 12.030 | H | N-$CH_3$ | $CH_3$ | 6-$CH_3$ | $CH_3$ | 1 | | |
| 12.031 | H | N-$CH_3$ | $CH_3$ | - | $CH_3$ | 1 | 185 Zers. | Na-Salz |
| 12.032 | H | N-$CH_3$ | $CH_3$ | - | $CH_3$ | 1 | 178 Zers. | |
| 12.033 | H | O | $C_2H_5$ | - | $CH_3$ | 0 | 141-144 | |
| 12.034 | H | O | $C_2H_5$ | - | $CH_3$ | 0 | 160 Zers. | Na-Salz |
| 12.035 | H | O | $C_2H_5$ | - | $CH_3$ | 1 | 127-130 | |
| 12.036 | $CH_3$ | O | $C_2H_5$ | - | $CH_3$ | 1 | | Na-Salz |
| 12.037 | H | O | $C_2H_5$ | 6-$CH_3$ | $CH_3$ | 0 | | |
| 12.038 | H | O | $C_2H_5$ | 5-Cl | $CH_3$ | 0 | 139-142 | |
| 12.039 | $CH_3$ | O | $C_2H_5$ | 5-Cl | $CH_3$ | 0 | | |
| 12.040 | H | O | $C_2H_5$ | 5-Cl | $CH_3$ | 1 | | |
| 12.041 | H | O | $CH_3$ | - | $C_2H_5$ | 0 | | |

Tabelle 12: (Fortsetzung)

| Nr. | $R^1$ | X | $R^2$ | $R^3$ | $R^5$ | n | Fp (°C) | |
|---|---|---|---|---|---|---|---|---|
| 12.042 | H | O | $CH_3$ | - | $C_2H_5$ | 0 | | Na-Salz |
| 12.043 | H | O | $CH_3$ | - | $C_2H_5$ | 1 | | |
| 12.044 | H | O | $C_2H_5$ | 4-Cl | $CH_3$ | 0 | 153 Zers. | Na-Salz |
| 12.045 | H | O | $CH_3$ | 6-Cl | $CH_3$ | 0 | 173-175 | |
| 12.046 | H | O | $CH_3$ | 3-Cl | $CH_3$ | 0 | | Na-Salz |
| 12.047 | H | O | $CH_3$ | 5-Cl | $CH_3$ | 0 | 144-145 | |
| 12.048 | H | O | $CH_3$ | 4-Cl | $CH_3$ | 0 | | Na-Salz |
| 12.049 | H | O | $CH_3$ | 3-Cl | $CH_3$ | 0 | 160 Zers. | |
| 12.050 | H | O | $CH_3$ | 4-Cl | $CH_3$ | 0 | 130-133 | |
| 12.051 | H | O | $C_2H_5$ | - | $CH_3$ | 1 | 155 Zers. | Na-Salz |
| 12.052 | H | N-$CH_3$ | $CH_3$ | 6-$CH_3$ | $CH_3$ | 0 | | |
| 12.053 | H | N-$CH_3$ | $CH_3$ | 3-$CH_3$ | $CH_3$ | 1 | 150 Zers. | |
| 12.054 | H | O | $C_2H_5$ | - | $C_2H_5$ | 0 | 139-143 Zers. | |
| 12.055 | H | NH | $CH_3$ | 3-$CH_3$ | $CH_3$ | 0 | 149-155 | |
| 12.056 | H | NH | $CH_3$ | 3-$CH_3$ | $CH_3$ | 1 | 149-155 | |
| 12.057 | H | N-$CH_3$ | $CH_3$ | 4-Cl | $CH_3$ | 0 | 165-169 | |
| 12.058 | H | N-$CH_3$ | $CH_3$ | 4-Cl | $CH_3$ | 0 | 159 Zers. | Na-Salz |
| 12.059 | H | N-$CH_3$ | $CH_3$ | 6-$CH_3$ | $CH_3$ | 0 | | |
| 12.060 | H | O | $C_2H_5$ | 4-Cl | $CH_3$ | 0 | 139-143 | |

Tabelle 12: (Fortsetzung)

| Nr. | $R^1$ | X | $R^2$ | $R^3$ | $R^5$ | n | Fp (°C) | |
|---|---|---|---|---|---|---|---|---|
| 12.061 | H | O | $C_2H_5$ | 4-Cl | $CH_3$ | 0 | 154 Zers. | Na-Salz |
| 12.062 | $CH_3$ | O | $CH_3$ | 4-Cl | $CH_3$ | 0 | 103-106 | |
| 12.063 | $CH_3$ | O | $CH_3$ | 4-Cl | $CH_3$ | 0 | 217 Zers. | Na-Salz |
| 12.064 | H | O | $CH_3$ | 3-Cl | $CH_3$ | 0 | 155 Zers. | Na-Salz |
| 12.065 | H | O | $CH_3$ | 4-Cl | $CH_3$ | 0 | 137 Zers. | Na-Salz |

Tabelle 13:

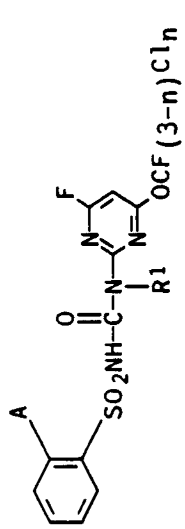

| Nr. | A | $R^1$ | n | Fp (°C) | |
|---|---|---|---|---|---|
| 13.001 | $CF_3$ | H | 1 | 179- 82 | |
| 13.002 | $CF_3$ | H | 1 | 144 | Na-Salz |
| 13.003 | $CF_3$ | H | 0 | 162-166 | |
| 13.004 | $CF_3$ | H | 0 | 107 | Na-Salz |
| 13.005 | Br | H | 0 | 138-142 | |
| 13.006 | Br | H | 0 | 149 Zers. | Na-Salz |

Anwendungsbeispiele

A Herbizide Wirkung

Die herbizide Wirkung der Sulfonylharnstoffe der Formel I ließ sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,015 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürz. | Lateinischer Name | Deutscher Name |
|---|---|---|
| AMARE | Amaranthus retroflexus | Zurückgekrümmter Fuchsschwanz |
| POLPE | Polygonum persicaria | Flohknöterich |
| TRZAW | Triticum aestivum | Winterweizen |

Mit 0,015 kg/ha a.S. im Nachauflaufverfahren eingesetzt, lassen sich mit den Beispielen 6.019 breitblättrige unerwünschte Pflanzen sehr gut bekämpfen bei gleichzeitiger Verträglichkeit an Weizen.

B Bioregulatorische Wirkung

Als Vergleichssubstanz dienten in den jeweiligen Beispielen
2-Chlorethyltrimethylammoniumchlorid (CCC) = "A"
6,7-Dihydrodipyridol(1,2-$\alpha$:2',1'-c)pyridilium-Ion als Dibromid-Monohydratsalz (Diquat) = "B"

Beispiel B.1

Untersuchung auf wachstumsregulierende Wirkung mit Reiskeimlingen

Junge Reiskeimlinge (Sorte Bahia) wurden in einer Nährlösung kultiviert, die die jeweiligen Wirkstoffe in unterschiedlichen Konzentrationen enthielt. Nach 6 Tagen Kultur bei 25 °C im Dauerlicht wurde die Wirkstoffkonzentration bestimmt, die das Längenwachstum der zweiten Blattscheide um 50 % vermindert (= $KI_{50}$).

(Details bei: W. Rademacher und J. Jung, Berichte aus dem Fachgebiet Herbologie, Heft 24, pp. 127-134, Universität Hohenheim, 1983).

| Wirkstoff Nr. | $KI_{50}$ (molar) |
|---|---|
| 1.001 | $8,6 \times 10^{-6}$ |
| 3.001 | $2,2 \times 10^{-6}$ |
| "A" | $1,5 \times 10^{-2}$ |

Beispiel B.2

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente CCC ("A").

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen.

Tabelle B.2.1

Sommergerste, "Aramir"

Nachauflauf-Blattbehandlung

| Nr. d. chem. Beispiele | Konz. mg WS/Gef. | Wuchshöhen rel. |
|---|---|---|
| unbehandelt | - | 100 |
| "A" | 0,025 | 100 |
| | 0,1 | 100 |
| | 0,38 | 96,3 |
| | 1,5 | 93,3 |
| 3.001 | 0,025 | 100 |
| | 0,1 | 100 |
| | 0,38 | 100 |
| | 1,5 | 100 |
| 5.001 | 0,025 | 56,3 |
| | 0,1 | 47,4 |
| | 0,38 | 41,5 |
| | 1,5 | 38,5 |
| 5.003 | 0,025 | 74,1 |
| | 0,1 | 59,3 |
| | 0,38 | 47,4 |
| | 1,5 | 41,5 |
| 5.019 | 0,025 | 62,2 |
| | 0,1 | 47,4 |
| | 0,38 | 41,5 |
| | 1,5 | 41,5 |
| 2.001 | 0,025 | 100 |
| | 0,1 | 100 |
| | 0,38 | 100 |
| | 1,5 | 100 |
| 1.003 | 0,025 | 100 |
| | 0,1 | 100 |
| | 0,38 | 85,9 |
| | 1,5 | 56,3 |
| 3.003 | 0,025 | 100 |
| | 0,1 | 100 |
| | 0,38 | 97,8 |
| | 1,5 | 81,5 |

Tabelle B.2.2

Sommerweizen, "Ralle"

Nachauflauf-Blattbehandlung

| Nr. d. chem. Beispiele | Konz. mg WS/Gef. | Wuchshöhen rel. |
|---|---|---|
| unbehandelt | - | 100 |
| "A" | 0,025 | 100 |
| | 0,1 | 91,9 |
| | 0,38 | 87,4 |
| | 1,5 | 81,4 |
| 3.001 | 0,025 | 100 |
| | 0,1 | 100 |
| | 0,38 | 100 |
| | 1,5 | 94,9 |
| 5.001 | 0,025 | 51,2 |
| | 0,1 | 48,2 |
| | 0,38 | 48,2 |
| | 1,5 | 48,2 |
| 5.003 | 0,025 | 63,3 |
| | 0,1 | 51,2 |
| | 0,38 | 48,2 |
| | 1,5 | 48,2 |
| 5.019 | 0,025 | 51,2 |
| | 0,1 | 48,2 |
| | 0,38 | 48,2 |
| | 1,5 | 48,2 |
| 2.001 | 0,025 | 100 |
| | 0,1 | 100 |
| | 0,38 | 100 |
| | 1,5 | 100 |
| 1.003 | 0,025 | 100 |
| | 0,1 | 100 |
| | 0,38 | 93,4 |
| | 1,5 | 57,3 |
| 3.003 | 0,025 | 100 |
| | 0,1 | 100 |
| | 0,38 | 100 |
| | 1,5 | 85,9 |

Tabelle B.2.3

Sommergerste, "Aramir"

Nachauflauf-Blattbehandlung

| Nr. d. chem. Beispiele | Konz. mg WS/Gef. | Wuchshöhen rel. |
|---|---|---|
| unbehandelt | - | 100 |
| "A" | 0,025 | 100 |
| | 0,1 | 100 |
| | 0,38 | 96,3 |
| | 1,5 | 93,3 |
| 1.003 | 0,025 | 100 |
| | 0,1 | 100 |
| | 0,38 | 100 |
| | 1,5 | 66,1 |
| 3.003 | 0,025 | 100 |
| | 0,1 | 100 |
| | 0,38 | 100 |
| | 1,5 | 83,3 |
| 12.032 | 0,025 | 86,0 |
| | 0,1 | 47,6 |
| | 0,38 | 39,7 |
| | 1,5 | 37,0 |

Tabelle B.2.4

Sommerweizen, "Ralle"

Nachauflauf-Blattbehandlung

| Nr. d. chem. Beispiele | Konz. mg WS/Gef. | Wuchshöhen rel. |
|---|---|---|
| unbehandelt | - | 100 |
| "A" | 0,025 | 100 |
| | 0,1 | 91,9 |
| | 0,38 | 87,4 |
| | 1,5 | 81,4 |
| 1.003 | 0,025 | 100 |
| | 0,1 | 100 |
| | 0,38 | 87,0 |
| | 1,5 | 48,5 |
| 3.003 | 0,025 | 100 |
| | 0,1 | 100 |
| | 0,38 | 82,7 |
| | 1,5 | 59,9 |
| 12.032 | 0,025 | 88,4 |
| | 0,1 | 44,2 |
| | 0,38 | 42,8 |
| | 1,5 | 42,8 |

Beispiel B.3

Junge Baumwollpflanzen (Sorte Stoneville 825, Entwicklungsstadium: 5 bis 6 entwickelte Laubblätter) wurden unter Gewächshausbedingungen angezogen Tag/Nachttemperatur 25/18 °C, relat. Luftfeuchte 50 bis 70 %) und tropfnaß mit wäßrigen Aufbereitungen der angegebenen Wirkstoffe (unter Zusatz von 0,15 Gew.% des Fettalkoholalkoxylats Plurafac® LF 700, bezogen auf die Spritzbrühe) blattbehandelt. Die umgerechnete Wassermenge betrug 1000 l/ha. 6 Tage nach Wirkstoffapplikation wurde die Anzahl abgeworfener Blätter bestimmt und der Grad der Entblätterung in % zur Kontrolle angegeben. Bei den unbehandelten Kontrollpflanzen trat kein Blattfall auf.

| Nr. d. chem. Beispiele | Umgerechnete Aufwandmenge kg/ha | % Entblätterung |
|---|---|---|
| 5.003 | 0,5 | 33 |
| 5.019 | 0,5 | 32 |
| "B" | 0,5 | 47 |

## Patentansprüche

### Patentansprüche für folgende Vertragsstaaten : AT, BE, CH, DE, FR, GB, IT, LI, NL

1. Substituierte Sulfonylharnstoffe der allgemeinen Formel I

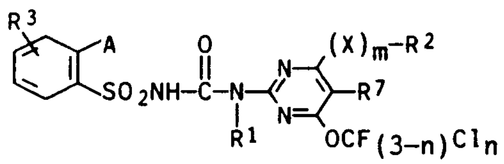

I,

in der n und m für 0 oder 1 stehen und die Substituenten folgende Bedeutung haben:

$R^1$ Wasserstoff, $C_1$-$C_4$-Alkyl, $C_3$-$C_6$-Alkenyl oder $C_3$-$C_6$-Alkinyl;

$R^2$ Halogen oder Trifluormethyl, wenn m für 0 steht oder $C_1$-$C_4$-Alkyl, $C_3$-$C_6$-Alkenyl oder $C_3$-$C_6$-Alkinyl, wenn m 1 bedeutet oder Trifluor-oder Chlordifluormethyl wenn X für 0 oder S und m für 1 steht;

X O, S oder N-$R^4$, wobei $R^4$ für Wasserstoff oder $C_1$-$C_4$-Alkyl steht;

$R^3$ Wasserstoff, Halogen, $C_1$-$C_4$-Alkyl, $C_1$-$C_4$-Halogenalkyl oder $C_1$-$C_4$-Alkoxy;

A $C_1$-$C_4$-Halogenalkyl, ein Halogenatom oder ein Rest,

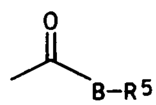

wobei

B ein Sauerstoffatom oder eine Alkyliminogruppe $NR^6$;

$R^5$ Wasserstoff, eine $C_1$-$C_6$-Alkylgruppe, welche bis zu drei der folgenden Reste tragen kann: Halogen, $C_1$-$C_4$-Alkoxy, $C_1$-$C_4$-Alkylthio, $C_1$-$C_4$-Halogenalkoxy, $C_1$-$C_4$-Alkoxy-$C_1$-$C_2$-alkoxy, $C_3$-$C_7$-Cycloalkyl und/oder Phenyl; eine $C_5$-$C_7$-Cycloalkylgruppe, welche bis zu drei $C_1$-$C_4$-Alkylgruppen tragen kann; eine $C_3$-$C_6$-Alkenylgruppe oder eine $C_3$-$C_6$-Alkinylgruppe;

$R^6$ Wasserstoff, eine $C_1$-$C_6$-Alkylgruppe, oder gemeinsam mit $R^5$ eine $C_4$-$C_6$-Alkylenkette, worin eine Methylengruppe durch ein Sauerstoffatom oder eine $C_1$-$C_4$-Alkyliminogruppe ersetzt sein kann, bedeutet,

$R^7$ Wasserstoff oder Halogen

sowie deren umweltverträgliche Salze.

2. Sulfonylharnstoffe nach Anspruch 1, in denen die Substituenten folgende Bedeutung haben:

$R^1$ Wasserstoff oder Methyl;

$R^2$ Halogen oder Trifluormethyl, wenn m für 0 steht und Methyl, wenn m 1 bedeutet;

X O oder NH;

$R^3$ Wasserstoff, Halogen oder Methyl und

A eine Gruppe $CO_2R^5$, worin $R^5$ $C_1$-$C_4$-Alkyl bedeutet;

$R^7$ Wasserstoff.

3. Herbizides Mittel, enthaltend einen Sulfonylharnstoff der Formel I gemäß Anspruch 1 oder sein Salz sowie hierfür übliche Trägerstoffe.

4. Verfahren zur Bekämpfung unerwünschten Pflanzenwuchses, dadurch gekennzeichnet, daß man einen Sulfonylharnstoff der Formel I gemäß Anspruch 1 oder eines seiner Salze in einer herbizid wirksamen Menge auf die Pflanzen und/oder ihren Lebensraum einwirken läßt.

5. Verfahren zur Bekämpfung unerwünschten Pflanzenwachstums in Getreide, dadurch gekennzeichnet, daß man eine herbizid wirksame Menge von 2-(((4-Methoxy-6-trifluormethoxy-1,3-pyrimidin-2-yl)-aminocarbonyl)-aminosulfonyl)-benzoesäuremethylester verwendet.

**6.** Verfahren zur Regulierung des Pflanzenwuchses, dadurch gekennzeichnet, daß man eine wachstumsregulierende Menge eines Sulfonylharnstoffes der Formel I gemäß Anspruch 1 oder eines seiner Salze auf die Samen, die Pflanzen und/oder deren Lebensraum einwirken läßt.

**7.** Verfahren zur Herstellung von Sulfonylharnstoffen der Formel I gemäß Anspruch 1, dadurch gekennzeichnet, daß man ein Sulfonylisocyanat II

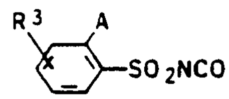

II

in an sich bekannter Weise in einem inerten organischen Lösungsmittel mit der ungefähr stöchiometrischen Menge eines substituierten 2-Amino-4-fluoralkoxy-pyrimidins der Formel III

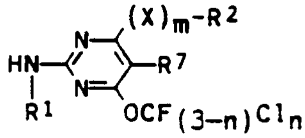

III

umsetzt, wobei die Substituenten und Indices die in Anspruch 1 angegebene Bedeutung haben und A nicht die Bedeutung COOH besitzt.

**8.** Verfahren zur Herstellung der Verbindungen I gemäß Anspruch 7, dadurch gekennzeichnet, daß man ein Carbamat der Formel IV

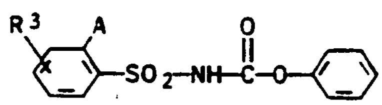

IV

in der A und $R^3$ die in Anspruch 1 angegebene Bedeutung haben, wobei A nicht die Bedeutung COOH besitzt, in an sich bekannter Weise in einem inerten organischen Lösungsmittel bei einer Temperatur zwischen 0 und 120 °C mit ungefähr der stöchiometrischen Menge eines 2-Amino-4-fluoralkoxypyrimidins III umsetzt.

**9.** Verfahren zur Herstellung von Sulfonylharnstoffen der Formel I gemäß Anspruch 7, dadurch gekennzeichnet, daß man ein entsprechendes Sulfonamid der Formel V

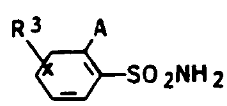

V

in an sich bekannter Weise in einem inerten organischen Lösungsmittel mit einem Phenylcarbamat VI

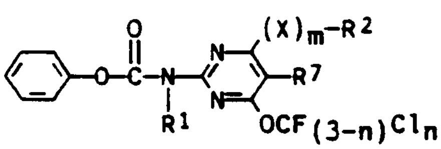

VI

umsetzt, wobei die Substituenten und Indices die in Anspruch 1 angegebene Bedeutung haben und A

nicht die Bedeutung COOH besitzt.

**10.** Substituierte 2-Amino-4-fluoralkoxy-pyrimidine der allgemeinen Formel IIIa

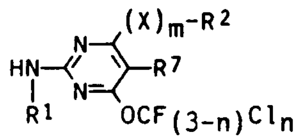

IIIa

in der m für 1 und n für 0 oder 1 steht und die Substituenten folgende Bedeutung haben:

$R^1$ Wasserstoff, $C_1$-$C_4$-Alkyl, $C_3$-$C_6$-Alkenyl oder $C_3$-$C_6$-Alkinyl;
$R^2$ $C_1$-$C_4$-Alkyl, $C_3$-$C_6$-Alkenyl oder $C_3$-$C_6$-Alkinyl;
X O, S oder N-$R^4$, wobei
$R^4$ für Wasserstoff oder $C_1$-$C_4$-Alkyl steht,
$R^7$ Wasserstoff oder Halogen.

**11.** Verfahren zur Herstellung von 2-Amino-4-fluoralkoxy-pyrimidinen der allgemeinen Formel IIIa gemäß Anspruch 10, dadurch gekennzeichnet, daß man 2-Amino-4-fluoralkoxy-6-halogen-pyrimidine der Formel IIIb

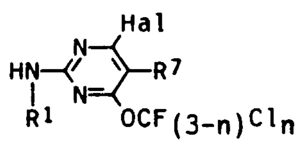

IIIb

in der Hal für Fluor, Chlor oder Brom steht und $R^1$, $R^7$ und n die vorgenannte Bedeutung haben, mit einem Nucleophil der Formel XVI

H-X-$R^2$ XVI

in der X und $R^2$ die vorgenannte Bedeutung besitzen, oder dessen Salz umsetzt.

**12.** 2-Amino-4-methoxy-6-trifluormethoxy-pyrimidin.

**13.** 2-Amino-4-methoxy-6-chlordifluormethoxy-pyrimidin.

**14.** 2-Amino-4-methylamino-6-trifluormethoxy-pyrimidin.

**15.** 2-Amino-4-methylamino-6-chlordifluormethoxy-pyrimidin.

**Patentansprüche für folgenden Vertragsstaat : ES**

**1.** Herbizide Mittel, enthaltend neben üblichen Trägerstoffen substituierte Sulfonylharnstoffe der allgemeinen Formel I

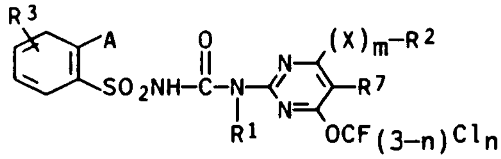

I,

in der n und m für 0 oder 1 stehen und die Substituenten folgende Bedeutung haben:

$R^1$ Wasserstoff, $C_1$-$C_4$-Alkyl, $C_3$-$C_6$-Alkenyl oder $C_3$-$C_6$-Alkinyl;
$R^2$ Halogen oder Trifluormethyl, wenn m für 0 steht oder $C_1$-$C_4$-Alkyl, $C_3$-$C_6$-Alkenyl oder $C_3$-$C_6$-Alkinyl, wenn m 1 bedeutet oder Trifluor-oder Chlordifluormethyl wenn X für 0 oder S und m

für 1 steht;

X O, S oder N-$R^4$, wobei $R^4$ für Wasserstoff oder $C_1$-$C_4$-Alkyl steht;

$R^3$ Wasserstoff, Halogen, $C_1$-$C_4$-Alkyl, $C_1$-$C_4$-Halogenalkyl oder $C_1$-$C_4$-Alkoxy;

A $C_1$-$C_4$-Halogenalkyl, ein Halogenatom oder ein Rest,

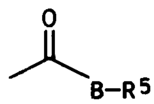

wobei

B ein Sauerstoffatom oder eine Alkyliminogruppe $NR^6$;

$R^5$ Wasserstoff, eine $C_1$-$C_6$-Alkylgruppe, welche bis zu drei der folgenden Reste tragen kann: Halogen, $C_1$-$C_4$-Alkoxy, $C_1$-$C_4$-Alkylthio, $C_1$-$C_4$-Halogenalkoxy, $C_1$-$C_4$-Alkoxy-$C_1$-$C_2$-alkoxy, $C_3$-$C_7$-Cycloalkyl und/oder Phenyl; eine $C_5$-$C_7$-Cycloalkylgruppe, welche bis zu drei $C_1$-$C_4$-Alkylgruppen tragen kann; eine $C_3$-$C_6$-Alkenylgruppe oder eine $C_3$-$C_6$-Alkinylgruppe;

$R^6$ Wasserstoff, eine $C_1$-$C_6$-Alkylgruppe, oder gemeinsam mit $R^5$ eine $C_4$-$C_6$-Alkylenkette, worin eine Methylengruppe durch ein Sauerstoffatom oder eine $C_1$-$C_4$-Alkyliminogruppe ersetzt sein kann, bedeutet,

$R^7$ Wasserstoff oder Halogen

sowie deren umweltverträgliche Salze.

**2.** Herbizide Mittel nach Anspruch 1, enthaltend Sulfonylharnstoffe der Formel I, in der die Substituenten folgende Bedeutung haben:

$R^1$ Wasserstoff oder Methyl;

$R^2$ Halogen oder Trifluormethyl, wenn m für 0 steht und Methyl, wenn m 1 bedeutet;

X O oder NH;

$R^3$ Wasserstoff, Halogen oder Methyl und

A eine Gruppe $CO_2R^5$, worin $R^5$ $C_1$-$C_4$-Alkyl bedeutet;

$R^7$ Wasserstoff.

**3.** Verfahren zur Bekämpfung unerwünschten Pflanzenwuchses, dadurch gekennzeichnet, daß man einen Sulfonylharnstoff der Formel I gemäß Anspruch 1 oder eines seiner Salze in einer herbizid wirksamen Menge auf die Pflanzen und/oder ihren Lebensraum einwirken läßt.

**4.** Verfahren zur Bekämpfung unerwünschten Pflanzenwachstums in Getreide, dadurch gekennzeichnet, daß man eine herbizid wirksame Menge von 2-(((4-Methoxy-6-trifluormethoxy-1,3-pyrimidin-2-yl)-aminocarbonyl)-aminosulfonyl)-benzoesäuremethylester verwendet.

**5.** Verfahren zur Regulierung des Pflanzenwuchses, dadurch gekennzeichnet, daß man eine wachstumsregulierende Menge eines Sulfonylharnstoffes der Formel I gemäß Anspruch 1 oder eines seiner Salze auf die Samen, die Pflanzen und/oder deren Lebensraum einwirken läßt.

**6.** Verfahren zur Herstellung von Sulfonylharnstoffen der Formel I gemäß Anspruch 1, in der A nicht die Bedeutung COOH besitzt, dadurch gekennzeichnet, daß man ein Sulfonylisocyanat II

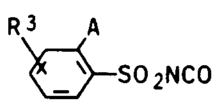

II

in an sich bekannter Weise in einem inerten organischen Lösungsmittel mit der ungefähr stöchiometrischen Menge eines substituierten 2-Amino-4-fluoralkoxy-pyrimidins der Formel III

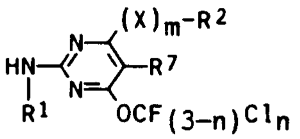

III

umsetzt, wobei die Substituenten und Indices die in Anspruch 1 angegebene Bedeutung haben und A nicht die Bedeutung COOH besitzt.

**7.** Verfahren zur Herstellung der Verbindungen I gemäß Anspruch 6, dadurch gekennzeichnet, daß man ein Carbamat der Formel IV

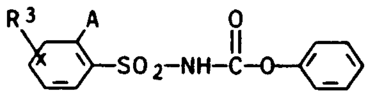

IV

in an sich bekannter Weise in einem inerten organischen Lösungsmittel bei einer Temperatur zwischen 0 und 120 °C mit ungefähr der stöchiometrischen Menge eines 2-Amino-4-fluoralkoxy-pyrimidins III umsetzt.

**8.** Verfahren zur Herstellung von Sulfonylharnstoffen der Formel I gemäß Anspruch 6, dadurch gekennzeichnet, daß man ein entsprechendes Sulfonamid der Formel V

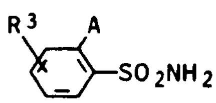

V

in an sich bekannter Weise in einem inerten organischen Lösungsmittel mit einem Phenylcarbamat VI

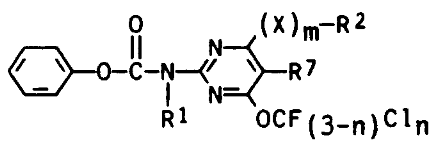

VI

umsetzt.

**9.** Verfahren zur Herstellung von 2-Amino-4-fluoralkoxy-pyrimidinen der allgemeinen Formel IIIa

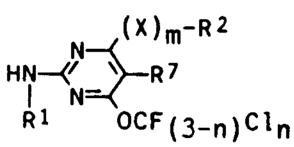

IIIa

in der m für 1 und n für O oder 1 steht und die Substituenten folgende Bedeutung haben:

- $R^1$ Wasserstoff, $C_1$-$C_4$-Alkyl, $C_3$-$C_6$-Alkenyl oder $C_3$-$C_6$-Alkinyl;
- $R^2$ $C_1$-$C_4$-Alkyl, $C_3$-$C_6$-Alkenyl oder $C_3$-$C_6$-Alkinyl;
- X O, S oder N-$R^4$, wobei
- $R^4$ für Wasserstoff oder $C_1$-$C_4$-Alkyl steht,
- $R^7$ Wasserstoff oder Halogen

dadurch gekennzeichnet, daß man 2-Amino-4-fluoralkoxy-6-halogen-pyrimidine der Formel IIIb

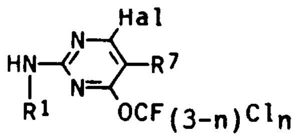

IIb

in der Hal für Fluor, Chlor oder Brom steht und $R^1$, $R^7$ und n die vorgenannte Bedeutung haben, mit einem Nucleophil der Formel XVI

H-X-$R^2$ XVI

in der X und $R^2$ die vorgenannte Bedeutung besitzen, oder dessen Salz umsetzt.

**Claims**

**Claims for the following Contracting States : AT, BE, CH, DE, FR, GB, IT, LI, NL**

**1.** A substituted sulfonylurea of the formula I

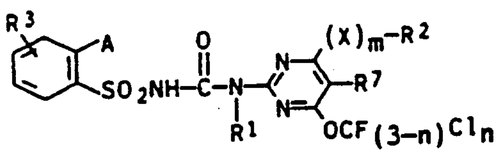

I,

where n and m are each 0 or 1, and

$R^1$ is hydrogen, $C_1$-$C_4$-alkyl, $C_3$-$C_6$-alkenyl or $C_3$-$C_6$-alkynyl,

$R^2$ is halogen or trifluoromethyl when m is 0, or $C_1$-$C_4$-alkyl, $C_3$-$C_6$-alkenyl or $C_3$-$C_6$-alkynyl when m is 1, or trifluoromethyl or chlorodifluoromethyl when X is O or S and m is 1,

X is O, S or N-$R^4$ where $R^4$ is hydrogen or $C_1$-$C_4$-alkyl,

$R^3$ is hydrogen, halogen, $C_1$-$C_4$-alkyl, $C_1$-$C_4$-haloalkyl or $C_1$-$C_4$-alkoxy,

A is $C_1$-$C_4$-haloalkyl, halogen or

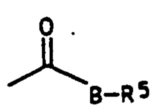

where

B is oxygen or alkylimino $NR^6$,

$R^5$ is hydrogen, $C_1$-$C_6$-alkyl which can carry up to three of the following: halogen, $C_1$-$C_4$-alkoxy, $C_1$-$C_4$-alkylthio, $C_1$-$C_4$-haloalkoxy, $C_1$-$C_4$-alkoxy-$C_1$-$C_2$-alkoxy, $C_3$-$C_7$-cycloalkyl and/or phenyl; or $C_5$-$C_7$-cycloalkyl which can carry up to three $C_1$-$C_4$-alkyl groups; or $C_3$-$C_6$-alkenyl or $C_3$-$C_6$-alkynyl,

$R^6$ is hydrogen, $C_1$-$C_6$-alkyl, or together with $R^5$ is $C_4$-$C_6$-alkylene in which one methylene can be replaced by oxygen or $C_1$-$C_4$-alkylimino,

$R^7$ is hydrogen or halogen, and environmentally tolerated salts thereof.

**2.** A sulfonylurea as claimed in claim 1, where

$R^1$ is hydrogen or methyl,

$R^2$ is halogen or trifluoromethyl when m is 0, and methyl when m is 1,

X is O or NH,

$R^3$ is hydrogen, halogen or methyl,

A is $CO_2R^5$ where $R^5$ is $C_1$-$C_4$-alkyl,

$R^7$ is hydrogen.

3. A herbicide containing a sulfonylurea of the formula I as claimed in claim 1, or a salt thereof, and conventional carriers therefor.

4. A method for controlling unwanted plant growth, wherein a herbicidally effective amount of a sulfonylurea of the formula I as claimed in claim 1, or a salt thereof, is allowed to act on the plants and/or their habitat.

5. A method for controlling unwanted plant growth in cereals, wherein a herbicidally effective amount of methyl 2-(((4-methoxy-6-trifluoromethoxy-1,3-pyrimidin-2-yl)aminocarbonyl)aminosulfonyl)benzoate is used.

6. A method for regulating plant growth, wherein a growth-regulating amount of a sulfonylurea of the formula I as claimed in claim 1, or a salt thereof, is allowed to act on the seeds, the plants and/or their habitat.

7. A process for preparing a sulfonylurea of the formula I as claimed in claim 1, wherein a sulfonyl isocyanate II

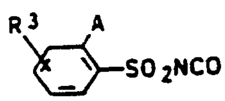

II

is reacted in a conventional manner in an inert organic solvent with approximately the stoichiometric amount of a substituted 2-amino-4-fluoroalkoxypyrimidine of the formula III

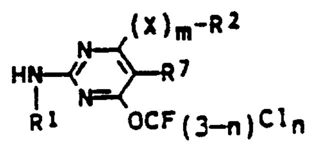

III

where the substituents and indices have the meanings specified in claim 1 and A is not COOH.

8. A process for preparing a compound I as claimed in claim 7, wherein a carbamate of the formula IV

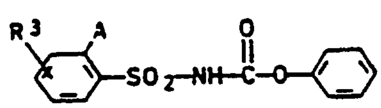

IV

where A and $R^3$ have the meanings specified in claim 1 and A is not COOH, is reacted in a conventional manner in an inert organic solvent at from 0 to 120 °C with approximately the stoichiometric amount of a 2-amino-4-fluoroalkoxypyrimidine III.

9. A process for preparing a sulfonylurea of the formula I as claimed in claim 7, wherein an appropriate sulfonamide of the formula V

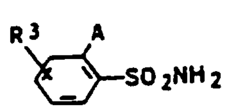

V

is reacted in a conventional manner in an inert organic solvent with a phenyl carbamate VI

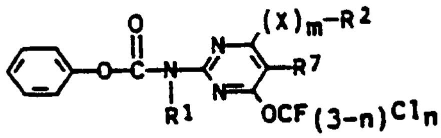

VI

where the substituents and indices have the meanings specified in claim 1 and A is not COOH.

**10.** A substituted 2-amino-4-fluoroalkoxypyrimidine of the formula IIIa

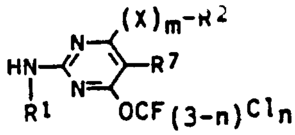

IIIa

where m is 1 and n is 0 or 1, and

$R^1$ is hydrogen, $C_1$-$C_4$-alkyl, $C_3$-$C_6$-alkenyl or $C_3$-$C_6$-alkynyl,
$R^2$ is $C_1$-$C_4$-alkyl, $C_3$-$C_6$-alkenyl or $C_3$-$C_6$-alkynyl,
X is O, S or N-$R^4$

where

$R^4$ is hydrogen or $C_1$-$C_4$-alkyl,
$R^7$ is hydrogen or halogen.

**11.** A process for preparing a 2-amino-4-fluoroalkoxypyrimidine of the formula IIIa as claimed in claim 10, wherein a 2-amino-4-fluoroalkoxy-6-halopyrimidine of the formula IIIb

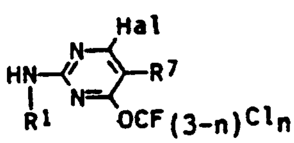

IIIb

where Hal is fluorine, chlorine or bromine and $R^1$, $R^7$ and n have the abovementioned meanings, is reacted with a nucleophile of the formula XVI

H-X-$R^2$ XVI

where X and $R^2$ have the abovementioned meanings, or a salt thereof.

**12.** 2-Amino-4-methoxy-6-trifluoromethoxypyrimidine.

**13.** 2-Amino-4-methoxy-6-chlorodifluoromethoxypyrimidine.

**14.** 2-Amino-4-methylamino-6-trifluoromethoxypyrimidine.

**15.** 2-Amino-4-methylamino-6-chlorodifluoromethoxypyrimidine.

**Claims for the following Contracting State : ES**

**1.** A herbicide containing, in addition to conventional carriers, a substituted sulfonylurea of the formula I

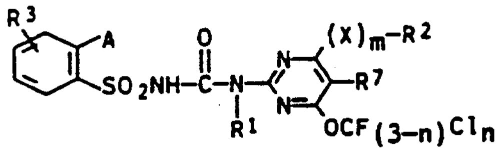

I,

where n and m are each 0 or 1, and

- $R^1$ is hydrogen, $C_1$-$C_4$-alkyl, $C_3$-$C_6$-alkenyl or $C_3$-$C_6$-alkynyl,
- $R^2$ is halogen or trifluoromethyl when m is 0, or $C_1$-$C_4$-alkyl, $C_3$-$C_6$-alkenyl or $C_3$-$C_6$-alkynyl when m is 1, or trifluoromethyl or chlorodifluoromethyl when X is O or S and m is 1,
- X is O, S or N-$R^4$ where $R^4$ is hydrogen or $C_1$-$C_4$-alkyl,
- $R^3$ is hydrogen, halogen, $C_1$-$C_4$-alkyl, $C_1$-$C_4$-haloalkyl or $C_1$-$C_4$-alkoxy,
- A is $C_1$-$C_4$-haloalkyl, halogen or

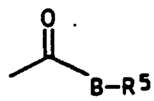

where

- B is oxygen or alkylimino $NR^6$,
- $R^5$ is hydrogen, $C_1$-$C_6$-alkyl which can carry up to three of the following: halogen, $C_1$-$C_4$-alkoxy, $C_1$-$C_4$-alkylthio, $C_1$-$C_4$-haloalkoxy, $C_1$-$C_4$-alkoxy-$C_1$-$C_2$-alkoxy, $C_3$-$C_7$-cycloalkyl and/or phenyl; or $C_5$-$C_7$-cycloalkyl which can carry up to three $C_1$-$C_4$-alkyl groups; or $C_3$-$C_6$-alkenyl or $C_3$-$C_6$-alkynyl,
- $R^6$ is hydrogen, $C_1$-$C_6$-alkyl, or together with $R^5$ is $C_4$-$C_6$-alkylene in which one methylene can be replaced by oxygen or $C_1$-$C_4$-alkylimino,
- $R^7$ is hydrogen or halogen,

and environmentally tolerated salts thereof.

**2.** A herbicide as claimed in claim 1, containing a sulfonylurea of the formula I, where

- $R^1$ is hydrogen or methyl,
- $R^2$ is halogen or trifluoromethyl when m is O, and methyl when m is 1,
- X is O or NH,
- $R^3$ is hydrogen, halogen or methyl,
- A is $CO_2R^5$ where $R^5$ is $C_1$-$C_4$-alkyl,
- $R^7$ is hydrogen.

**3.** A method for controlling unwanted plant growth, wherein a herbicidally effective amount of a sulfonylurea of the formula I as claimed in claim 1, or a salt thereof, is allowed to act on the plants and/or their habitat.

**4.** A method for controlling unwanted plant growth in cereals, wherein a herbicidally effective amount of methyl 2-(((4-methoxy-6-trifluoromethoxy-1,3-pyrimidin-2-yl)aminocarbonyl)aminosulfonyl)benzoate is used.

**5.** A method for regulating plant growth, wherein a growth-regulating amount of a sulfonylurea of the formula I as claimed in claim 1, or a salt thereof, is allowed to act on the seed, the plants and/or their habitat.

**6.** A process for preparing a sulfonylurea of the formula I as claimed in claim 1, where A is not COOH, wherein a sulfonyl isocyanate II

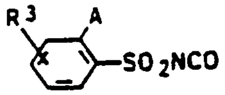

II

is reacted in a conventional manner in an inert organic solvent with approximately the stoichiometric amount of a substituted 2-amino-4-fluoroalkoxypyrimidine of the formula III

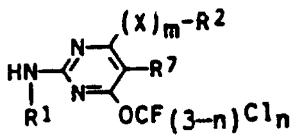

III

where the substituents and indices have the meanings specified in claim 1 and A is not COOH.

**7.** A process for preparing a compound I as claimed in claim 6, wherein a carbamate of the formula IV

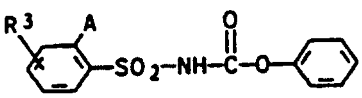

IV

is reacted in a conventional manner in an inert organic solvent at from 0 to 120 °C with approximately the stoichiometric amount of a 2-amino-4-fluoroalkoxypyrimidine III.

**8.** A process for preparing a sulfonylurea of the formula I as claimed in claim 6, wherein an appropriate sulfonamide of the formula V

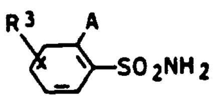

V

is reacted in a conventional manner in an inert organic solvent with a phenyl carbamate VI

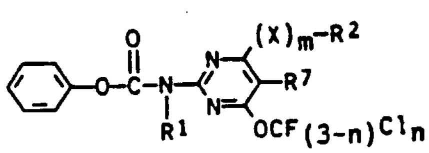

VI

**9.** A process for preparing a 2-amino-4-fluoroalkoxypyrimidine of the formula IIIa

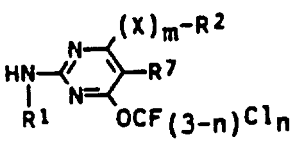

IIIa

where m is 1 and n is 0 or 1, and

- $R^1$ is hydrogen, $C_1$-$C_4$-alkyl, $C_3$-$C_6$-alkenyl or $C_3$-$C_6$-alkynyl,
- $R^2$ is $C_1$-$C_4$-alkyl, $C_3$-$C_6$-alkenyl or $C_3$-$C_6$-alkynyl,
- X is O, S or N-$R^4$ where

$R^4$ is hydrogen or $C_1$-$C_4$-alkyl,
$R^7$ is hydrogen or halogen,
wherein a 2-amino-4-fluoroalkoxy-6-halopyrimidine of the formula IIIb

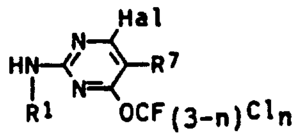

IIIb

where Hal is fluorine, chlorine or bromine and $R^1$, $R^7$ and n have the abovementioned meanings, is reacted with a nucleophile of the formula XVI

H-X-$R^2$ XVI

where X and $R^2$ have the abovementioned meanings, or a salt thereof.

**Revendications**
**Revendications pour les Etats contractants suivants : AT, BE, CH, DE, FR, GB, IT, LI, NL**

**1.** Sulfonylurées substituées, de formule générale I,

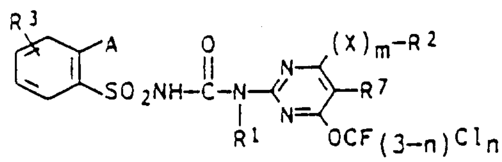

I,

dans laquelle n et m sont mis pour 0 ou 1 et les substituants ont les significations suivantes :
$R^1$ hydrogène, alkyle en C1-C4, alcényle en C3-C6 ou alcynyle en C3-C6 ;
$R^2$ halogène ou trifluorométhyle lorsque m est mis pour 0 ou alkyle en C1-C4, alcényle en C3-C6 ou alcynyle en C3-C6 lorsque m représente 1, ou trifluoro- ou chlorodifluorométhyle lorsque X est mis pour O ou S et m pour 1 ;
X O, S ou N-R4, $R^4$ étant mis pour hydrogène ou alkyle en C1-C4 ;
$R^3$ hydrogène, halogène, alkyle en C1-C4, halogénoalkyle en C1-C4 ou alcoxy en C1-C4 ;
A hélogénoalkyle en C1-C4, un atome d'halogène ou un reste

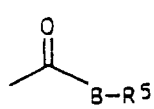

où
B est un atome d'oxygène ou un groupe alkylimino $NR^6$ ;
$R^5$ représente hydrogène, un groupe alkyle en C1-C6 pouvant porter jusqu'à trois des restes halogène, alcoxy en C1-C4, alkylthio en C1-C4, halogénoalcoxy en C1-C4, alcoxy en C1-C4-alcoxy en C1-C2, cycloalkyle en C3-C7 et/ou phényle ; un groupe cycloalkyle en C5-C7 pouvant porter jusqu'à trois groupes alkyle en C1-C4 ; un groupe alcényle en C3-C6 ou un groupe alcynyle en C3-C6 ;
$R^6$ représente hydrogène, un groupe alkyle en C1-C6 ou, ensemble avec $R^5$, une chaîne alkylène en C4-C6 dans laquelle un groupe méthylène peut être remplacé par un atome d'oxygène ou un groupe alkylimino en C1-C4 ;
$R^7$ hydrogène ou halogène :,
ainsi que leurs sels acceptables pour l'environnement.

**2.** Sulfonylurées selon la revendication 1, dans lesquelles les substituants ont les significations suivantes :
$R^1$ hydrogène ou méthyle ;
$R^2$ halogène ou trifluorométhyle lorsque m est mis pour 0 et méthyle lorsque m représente 1 ;

X O ou NH ;
$R^3$ hydrogène, halogène ou méthyle et
A un groupe $CO_2R^5$ dans lequel $R^5$ représente un groupe alkyle en C1-C4 ;
$R^7$ hydrogène.

**3.** Agent herbicide contenant une sulfonylurée de formule I, selon la revendication 1, ou son sel, ainsi que des véhicules usuels respectifs.

**4.** Procédé de lutte contre la croissance de plantes indésirables, caractérisé par le fait que l'on fait agir sur les plantes et/ou leur biotope une quantité efficace au point de vue herbicide d'une sulfonylurée de formule I, selon la revendication 1, ou d'un de ses sels.

**5.** Procédé de lutte contre la croissance de plantes indésirables dans les céréales, caractérisé par le fait que l'on utilise une quantité à efficacité herbicide d'un 2-(((4-méthoxy-6-trifluorométhyl-1,3-pyrimidin-2-yl)-aminocarbonyl)-aminosulfonyl)-benzoate de méthyle.

**6.** Procédé de régulation de la croissance des plantes, caractérisé par le fait que l'on fait agir sur les les plantes et/ou leur biotope une quantité, susceptible de réguler leur croissance, d'une sulfonylurée de formule I, selon la revendication 1, ou d'un de ses sels.

**7.** Procédé de préparation de sulfonylurées de formule I, selon la revendication 1, caractérisé par le fait que l'on fait réagir un sulfonylisocyanate II

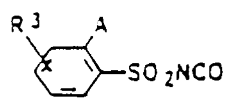

II

de manière, en soi, connue, dans un solvant organique inerte, avec une quantité environ stoechiométrique d'une 2-amino-4-fluoralcoxypyrimidine de formule III

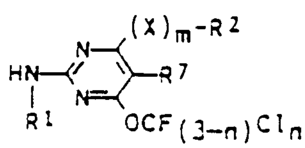

III

les substituants et les indices ayant les significations données dans la revendication 1 et A ne représentant pas COOH.

**8.** Procédé de préparation des composés I, selon la revendication 7, caractérisé par le fait que l'on fait réagir de manière, en soi, connue, dans un solvant organique inerte, à une température comprise entre 0 et 120 °C, un carbamate de formule IV

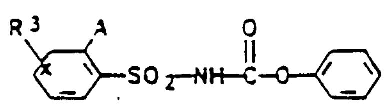

IV

dans laquelle A et $R^3$ ont les significations données dans la revendication 1, A ne représentant pas COOH, avec une quantité environ stoechiométrique d'une 2-amino-4-fluoralcoxypyrimidine III.

**9.** Procédé de préparation de sulfonylurées de formule I, selon la revendication 7, caractérisé par le fait que l'on fait réagir de manière, en soi, connue, dans un solvant organique inerte, un sulfonamide approprié de formule V

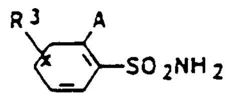

V

avec un phénylcarbamate de formule VI

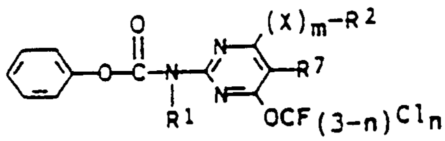

VI

les substituants et indices ayant les significations données dans la revendication 1 et A ne représentant pas COOH.

**10.** 2-amino-4-fluoralcoxy-pyrimidines substituées, de formule générale IIIa

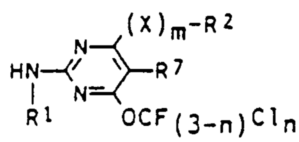

IIIa

dans laquelle m est mis pour 1 et n pour 0 ou 1 et les substituants ont les significations suivantes :
$R^1$ hydrogène, alkyle en C1-C4, alcényle en C3-C6 ou alcynyle en C3-C6 ;
$R^2$ alkyle en C1-C4, alcényle en C3-C6 ou alcynyle en C3-C6 ;
X O, S ou N-$R^4$ où
$R^4$ est mis pour hydrogène ou alkyle en C1-C4,
$R^7$ hydrogène ou halogène.

**11.** Procédé de préparation de 2-amino-4-fluoralcoxy-pyrimidines de formule générale IIIa, selon la revendication 10, caractérisé par le fait que l'on fait réagir des 2-amino-4-fluoralcoxy-6-halogéno-pyrimidines de formule IIIb

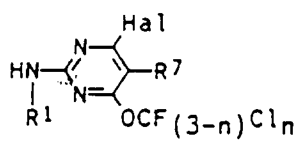

IIIb

dans laquelle Hal est mis pour fluor, chlore ou brome et $R^1$, $R^7$ et n ont les significations données plus haut, avec un nucléophile de formule XVI

H-X-$R^2$ XVI

dans laquelle X et $R^2$ ont les significations données plus haut, ou avec son sel.

**12.** 2-amino-4-méthoxy-6-trifluorométhoxy-pyrimidine.

**13.** 2-amino-4-méthoxy-6-chlorodifluorométhoxy-pyrimidine.

**14.** 2-amino-4-méthylamino-6-trifluorométhoxy-pyrimidine.

**15.** 2-amino-4-méthylamino-6-chlorodifluorométhoxy-pyrimidine.

**Revendications pour l'Etat contractant suivant : ES**

1. Produit herbicide contenant, outre des véhicules usuels, des sulfonylurées substituées, de formule générale I,

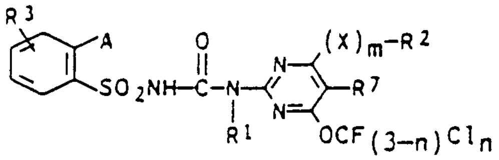

I,

dans laquelle n et m sont mis pour 0 ou 1 et les substituants ont les significations suivantes :
$R^1$ hydrogène, alkyle en C1-C4, alcényle en C3-C6 ou alcynyle en C3-C6 ;
$R^2$ halogène ou trifluorométhyle lorsque m est mis pour 0 ou alkyle en C1-C4, alcényle en C3-C6 ou alcynyle en C3-C6 lorsque m représente 1, ou trifluoro- ou chlorodifluorométhyle lorsque X est mis pour O ou S et m pour 1 ;
X O, S ou N-R4, $R^4$ étant mis pour hydrogène ou alkyle en C1-C4 ;
$R^3$ hydrogène, halogène, alkyle en C1-C4, halogénoalkyle en C1-C4 ou alcoxy en C1-C4 ;
A hélogénoalkyle en C1-C4, un atome d'halogène ou un reste

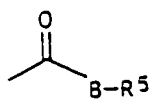

où
B est un atome d'oxygène ou un groupe alkylimino $NR^6$ ;
$R^5$ représente hydrogène, un groupe alkyle en C1-C6 pouvant porter jusqu'à trois des restes halogène, alcoxy en C1-C4, alkylthio en C1-C4, halogénoalcoxy en C1-C4, alcoxy en C1-C4-alcoxy en C1-C2, cycloalkyle en C3-C7 et/ou phényle ; un groupe cycloalkyle en C5-C7 pouvant porter jusqu'à trois groupes alkyle en C1-C4 ; un groupe alcényle en C3-C6 ou un groupe alcynyle en C3-C6 ;
$R^6$ représente hydrogène, un groupe alkyle en C1-C6 ou, ensemble avec $R^5$, une chaîne alkylène en C4-C6 dans laquelle un groupe méthylène peut être remplacé par un atome d'oxygène ou un groupe alkylimino en C1-C4 ;
$R^7$ hydrogène ou halogène :,
ainsi que leurs sels acceptables pour l'environnement.

2. Produit herbicide selon la revendication 1 contenant des sulfonylurées de la formule I, dans laquelle les substituants ont les significations suivantes :
$R^1$ hydrogène ou méthyle ;
$R^2$ halogène ou trifluorométhyle lorsque m est mis pour 0 et méthyle lorsque m représente 1 ;
X O ou NH ;
$R^3$ hydrogène, halogène ou méthyle et
A un groupe $CO_2R^5$ dans lequel $R^5$ représente un groupe alkyle en C1-C4 ;
$R^7$ hydrogène.

3. Procédé de lutte contre la croissance de plantes indésirables, caractérisé par le fait que l'on fait agir sur les plantes et/ou leur biotope une quantité efficace au point de vue herbicide d'une sulfonylurée de formule I, selon la revendication 1, ou d'un de ses sels.

4. Procédé de lutte contre la croissance de plantes indésirables dans les céréales, caractérisé par le fait que l'on utilise une quantité à efficacité herbicide d'un 2-(((4-méthoxy-6-trifluorométhyl-1,3-pyrimidin-2-yl)-aminocarbonyl)-aminosulfonyl)-benzoate de méthyle.

5. Procédé de régulation de la croissance des plantes, caractérisé par le fait que l'on fait agir sur les semences, les plantes et/ou leur biotope une quantité, susceptible de réguler leur croissance, d'une

sulfonylurée de formule I, selon la revendication 1, ou d'un de ses sels.

6. Procédé de préparation de sulfonylurées de formule I, selon la revendication 1, caractérisé par le fait que l'on fait réagir un sulfonylisocyanate II

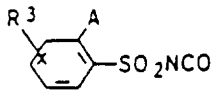

II

de manière, en soi, connue, dans un solvant organique inerte, avec une quantité environ stoechiométrique d'une 2-amino-4-fluoralcoxypyrimidine de formule III

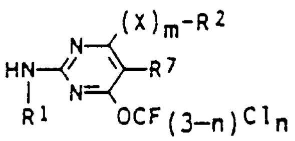

III

les substituants et les indices ayant les significations données dans la revendication 1 et A ne représentant pas COOH.

7. Procédé de préparation des composés I, selon la revendication 6, caractérisé par le fait que l'on fait réagir de manière, en soi, connue, dans un solvant organique inerte, à une température comprise entre 0 et 120 ° C, un carbamate de formule IV

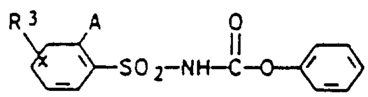

IV

avec une quantité environ stoechiométrique d'une 2-amino-4-fluoralcoxypyrimidine III.

8. Procédé de préparation de sulfonylurées de formule I, selon la revendication 6, caractérisé par le fait que l'on fait réagir de manière, en soi, connue, dans un solvant organique inerte, un sulfonamide approprié de formule V

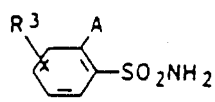

V

avec un phénylcarbamate VI

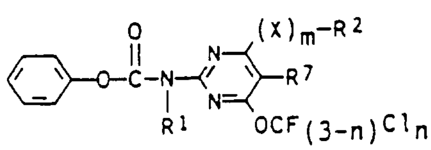

VI

**9.** Procédé de préparation de 2-amino-4-fluoralcoxy-pyrimidines de formule générale IIIa

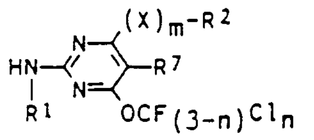

IIIa

dans laquelle m est mis pour 1 et n pour 0 ou 1 et les substituants ont les significations suivantes :
$R^1$ hydrogène, alkyle en C1-C4, alcényle en C3-C6 ou alcynyle en C3-C6 ;
$R^2$ alkyle en C1-C4, alcényle en C3-C6 ou alcynyle en C3-C6 ;
X O, S ou N-$R^4$, où :
$R^4$ représente hydrogène ou alkyle en C1-C4,
$R^7$ hydrogène ou halogène
caractérisé par le fait que l'on fait réagir des 2-amino-4-fluoralcoxy-6-halogéno-pyrimidines de formule IIIb

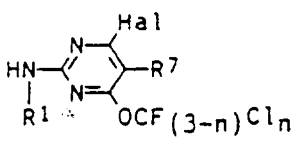

IIIb

dans laquelle Hal est mis pour fluor, chlore ou brome et $R^1$, $R^7$ et n ont les significations données plus haut, avec un nucléophile de formule XVI

H-X-$R^2$ XVI

dans laquelle X et $R^2$ ont les significations données plus haut, ou avec son sel.